# EUROPEAN PATENT APPLICATION

(11) **EP 1 795 204 A1**
(43) Date of publication of application: **13.06.2007**
(21) Application number: 05788363.9
(22) Date of filing: 27.09.2005
(51) Int. Cl.: A61K 39/395

(54) **FUNCTIONAL COMPOSITION OR FOOD CONTAINING WHEY PROTEIN, ANTIBODY DERIVED FROM MILK, OR ANTIBODY**

(30) Priority: 29.09.2004 JP 2004283388; 29.09.2004 JP 2004283389; 29.10.2004 JP 2004316331; 29.10.2004 JP 2004316332; 12.11.2004 JP 2004329801; 01.12.2004 JP 2004348131; 09.12.2004 JP 2004357105; 28.02.2005 JP 2005053074; 16.08.2005 JP 2005236072; 06.09.2005 JP 2005258260
(71) Applicant: Asama Chemical Co., Ltd., Tokyo 1030001 (JP); Shizuoka Prefecture, Shizuoka-shi Shizuoka 420-8601 (JP)
(72) Inventor: YAJIMA, Mizuo, c/o Asama Chemical Co., Ltd., Tokyo 1030001 (JP); IWATSUKI, Satoshi, c/o Asama Chemical Co., Ltd., Tokyo 1030001 (JP); SHIONOYA, Hiroshi, c/o Asama Chemical Co., Ltd., Tokyo 1030001 (JP); TERADO, Kuniaki, c/o Asama Chemical Co., Ltd., Tokyo 1030001 (JP); AKAMATSU, Hirohisa, Fujinomiya-shi, Shizuoka 4180005 (JP); SUZUKI, Shuuka, Hamamatsu-shi, Shizuoka 4300803 (JP); KATOH, Masamichi, Shizuoka-shi, Shizuoka 4200881 (JP)
(74) Representative: Ackroyd, Robert
(86) International application number: PCT/JP2005/018249
(87) International publication number: WO 2006/035979

(57) **Abstract**

A functional composition or food containing whey protein, an antibody derived from milk, or an antibody; and a whey protein food characterized by comprising whey protein and any of a glucide, cocoa powder, powdery non-coagulable insoluble, powdered green tea, aloe powder, turmeric powder, pumpkin powder, red grape juice powder, tomato powder, cranberry powder, raspberry powder, blueberry powder, and or strawberry powder.

## Description

### Technical Field

The present invention relates to a functional composition or food comprising whey protein, antibody derived from milk or antibody.

### Background Art

Rheumatoid arthritis is an inflammatory joint disease starting from flare, pain and swelling in arm and leg joints, proceeding to destruction and deformation of joints, and reaching to functional disorder in joints along with pain. For the pharmacotherapy, a conbination medication of an adrenocorticotropic hormonal agent, an immunosuppressant, an acidic anti-inflammatory agent, an organic gold compound, an anti-cytokine antibody and the other agents are medicated to suppress inflammation. The combination medication for pharmacotherapy is, however, nothing but a palliative treatment.

Today, rheumatoid arthritis is pathologically unobvious, and therefore researches have been made to have the base on a doctrine that it is associated with autoimmunity against Type II collagen which is a main component of articular cartilage.

Though many antigenic substances are found in food, the food is usually ingested to bring no antibodies against such antigens. This phenomenon is called tolerance to oral immunity.

An antibody production is regulated by one lymphocyte (helper T cell, abbreviated as Th) which functions to help the production and another lymphocyte (suppressor T cell, abbreviated as Ts) which functions to suppress. The suppressor T cell in a dominant state inhibits the antibody production. In order to inhibit production of the autoantibody to Type II collagen, a peptide from the Type II collagen from an animal or a denatured product of the Type II collagen is attempted to administer to activate the suppressor T cell against the collagen. The attempt includes JP-A 07-138187, JP-A 09-059176, and JP-A 09-255581, and they are all leading technologies for tolerance to oral immunity.

The present inventors have made a pathological research on rheumatoid arthritis to prevent and treat the disease. The research was initiated based on a hypothesis that rheumatoid arthritis occurs from the internal factor that is a dysfunction in oral immunity caused by a constitutional or genetical factor seen in a patient with rheumatoid arthritis, and the external factor that is a heterogenous Type II collagen or a bacterial endotoxin absorbed from a food through the gastrointestinal tract.

The dysfunction in oral immunity refers to the following state. Tolerance in oral immunity functions with a normal immune response against an ingested antigen, but internal, constitutional, environmental factors would interrupt the induction of the normal immune tolerance, resulting in the production of autoantibody. To examine this hypothesis in relation to mouse collagen-induced arthritis, mice were used to investigate the activity of the similar antigen and a bacterial endotoxin to induce arthritis. A DBA/1 mouse has a genetic trait for easily bringing about Type II collagen-induced arthritis. Oral administration of chicken-derived or heat-denatured Type II collagen to the mice produced mouse Type II collagen antibody in their blood to induce arthritis. This arthritis was aggravated by administration of the combination with bacterial endotoxin (also known as lipopolysaccharide or LPS). Further, long-term administration of bacterial endotoxin alone, wherein chicken-derived Type II collagen was removed, also induced arthritis (K. Terato et al. Br. J. Rheum. 35, 828-838, 1996).

These results show that along with anti-Type II collagen antibody, the bacterial endotoxin activates or exhausts non-specifically immune system to involve itself in the induction of rheumatoid arthritis, and further oral immunity tolerance sometimes does not function. This experiment also showed that heat- denatured Type II collagen could not induce immunity tolerance though it was prepared to induce immunity tolerance as shown in the above described JP-A 07-138187, JP-A 09-059176 and JP-A 09-255581 were exhibited.

Gastrointestinal immunity is positioned on the front line of immune system, and gastrointestinal tract gathers 70% of immune systems in a whole body. Enhanced gastrointestinal immunity could strengthen the immune system in the whole body. Strengthened immune system would increase the amount of an antibody secreted in gastrointestinal tract to prevent a bad bacteria from proliferation, resulting in improved intestinal environment, that is, a good intestinal condition provided. Further, the improved intestinal environment would activate gastrointestinal immunity. In this way, immunomodulatory effects and intestine-regulating effects associate with each other, so that they may be improved respectively and influenced mutually by a better effect to give a healthier body.

By the way, the antibody produced inside the body decreases in amount with aging to arm the elderly with lowered immune function. Further, a stress or an immunosuppressive pharmaceutical used may likewise reduce antibody production.

An antibody derived from milk can be ingested to enhance gastrointestinal immunity as positioned on the front line of immune system, thereby promoting the health, facilitating the recovery from disease, and promising a long and healthy life. For example, it is reported that human and animals can ingest the antibody derived from milk to treat and prevent effectively diseases caused by bacteria and viruses in gastrointestinal tract and to keep microorganisms in their normal balance in gastrointestinal tract, resulting in promoting the health (Korhonen H. et al. Bovine milk antibodies for health, British J. Nutrition, 84, suppl.1, S135-S146 (2000)). Further, it has been known that E. coli and the other Gram-negative bacteria, which reside in gastrointestinal tract, can release endotoxin as their body component into an animal body to induce a severe syndrome such as endotoxin shock and thrombosis. It, however, is reported that the antibody derived from milk can be ingested prior to surgery to eliminate the endotoxin's toxicity and to prevent damages by endotoxin (Bolke E, et al. Shock 17, 9-12 (2002)).

In an adult, an antibody is synthesized at a rate of 24 mg/day per kg of body mass to secrete on the mucosa of a gastrointestinal or respiratory tract (William E. Paul ed./Tomio Tada, translation supervisor, Basic Immunology (Tokyo University Press) 1986, Upper vol., p.181), thus approximately 1000 mg of antibody is secreted daily in the gastrointestinal tract, so that the adult should ingest antibody to supplement with some percentage of this amount in order to recovery from the disease and promotion of the health.

Usually, immunity established by the administration of antibody from outside body is referred to as passive immunity, while immunity established by producing antibody inside the body as active immunity. The ingestion of antibody described above belongs to passive immunity.

Meanwhile, conventional immunostimulatory substances activate active immunity. For example, it is known that so called mushrooms such as Agaricus, Meshimakobu, Enokidake, Hiratake, Nameko and Matutake and yeasts such as bread yeast, beer yeast and torula yeast contain β-glucan, thereby showing such activating effect. Further, some yeasts contain much glutathione, which also has immunostimulatory action. Bacteria such as lactobacilli and bifidobacteria and levansucrase can be ingested viably as probiotics to provide immunostimulatory and intestine-regulating effects. Further, bacteria can be ingested as dead cells to give similar effects, and the active component is known to be peptidoglycan which is a component for the cell wall. The present inventors have already demonstrated that the cell wall is decomposed by an enzyme to strengthen the immunostimulatory effect (JP-A 2000-4830, JP-A 2000-210050). The present inventors have already demonstrated that the DNA (deoxyribonucleic acid) derived from bacteria also can be ingested to provide the immunostimulatory effect (JP-A 2000-262247).

However, these bacteria give extremely their varying effects depending on ingestion methods and performance of an individual. In addition, it is also known that the immunostimulatory effects given by glutathione, lactoferrin and the like are not sufficient to use. A combination of immunostimulatory substances which promote active immunity is ingested to give no expectable synergistic effect because an individual has a limit in ability to produce antibody. In particular, the elderly, who has weakened ability to produce antibody, can not ingest to obtain so large an effect. Therefore, a functional composition having stronger immunomodulatory and intestine-regulating effect has strongly been desired to produce.

It has been known that lactobacilli and bifidobacteria have such effects as intestine-regulating effect, blood cholesterol- and blood pressure-lowering effect, anticancer effect, and further immunostimulatory effect (Toshiaki Takano et al., Science and technology of Lactic Acid Bacteria, p.311-334 (1996), Japan Scientific Academy Press) . They can be contained in a food by at least 10⁷/g or more to give expectable immunostimulatory effect. Further, lactobacilli or/and bifidobacteria can be contained in a food by 10⁷/g or more to give acknowledgeable effect for inhibiting other saprophytic bacteria, in particular, food poisoning bacteria from proliferation.

Many foods such as yogurt contain lactobacilli or bifidobacteria, but there has not been available a food which has a combination of the function possessed by lactobacilli or bifidobacteria with a function provided by an antibody.

Whey protein is similar to human milk in amino acid sequence, has a high nutritive value, contains all essential amino acids, can be absorbed at an extremely high rate and has a high content of branched amino acids, so that it has been commercialized as a dietary supplement to supply proteins mainly for an athlete and a person loaded with a high exercise. However, whey protein has a problem in flavor (JP-A 2003-23963), and thus the supplement keeps milk smell, puckery, and astringent tastes provided by whey protein. Therefore, a flavor such as vanilla is added in some products to improve for easy ingestion, but the tastes are only masked by the strong flavor to remain. Further, whey protein is added, for example, into water or cow milk to give easily "floating masses" due to its poor water absorption.

An adult secrets an immune antibody by an amount of more than 1000 mg/day into the gastrointestinal tract, but does it by a decreased amount with aging over the middle age. Therefore, it is extremely useful for the elderly to ingest an antibody-containing whey protein in order to maintain the health. However, there has not yet commercialized a food made of whey protein which the elderly can ingest to enjoy tasting everyday.

For decreasing milk smell, there have been proposed methods such as a method by JP-A 11-178506 wherein dihydrochalcones are added for improving the flavor of powdered milk or food and drink prepared from powdered milk, a method by of JP-A 2002-2531413 and JP-A 2002-2531644 wherein phosphorylated sugars, phosphorylated oligosaccharides or their mineral compounds are added for improving the milk feeling of milk and food containing powdered milk, a method by JP-A 2002-335903 wherein β-gluco-oligosaccharides are added for improving taste of food and drink containing milk, and a method by JP-A 2003-210119 wherein chlorogenic acid is added for inhibiting smell of protein of milk or animals. However, these methods achieve nothing but reducing milk smell or changing to cow milk-like flavor.

Further, there has been proposed a method wherein an α-binding galacto-oligosaccharide is added for decreasing astringency (JP-A 2003-250486), but this method is mainly intended to decrease astringency and bitterness in juice, coffee and beer, and does not relate to astringency of whey protein.

Among dairy products, whey protein can be expected to allow an antibody to be transferred therein to have an increased content. However, the pure whey protein contains an antibody at a rate of 10% by mass (Tamotsu Kuwata, Monthly Food Chemical, 7 (2), 68-77 (1991), WPC, which has a protein content of 80% by mass, can contain 8.0% by mass of an antibody as transferred without being inactivated), while a commercially available whey proteins does not contains so much. The commercially available whey protein WPC (wheyprotein concentrate, protein content 80% by mass) has a maximal antibody content of 5.5% by mass, and a WPI (whey protein isolate, protein content 90% by mass) has that of 2.4% by mass, because 30% or more of the antibody is heated to inactivate in the production process. Further, a commercially available IgG concentration whey protein, which contains an antibody by 10% by mass or more, is extremely expensive, because it takes a larger chromatography apparatus for the antibody to be fractionated from a whey protein of a smaller amount treated per hour. If a current apparatus can be used to produce a WPC or a WPI which has a higher antibody content than the conventional whey protein product, it would facilitate to enjoy antibody functions described above.

Many powdered proteins derived from milk are dispersed in water to bring about a "clump", and difficult to get uniformly dispersed. Namely, they are apt to form a "floating mass" which is hardly dispersible. It takes a strong and long-time stirring to disperse the mass uniformly.

For improving water dispersibility, there is proposed a method wherein a hardly dispersible food is kneaded with an aqueous solution of monosaccharide, disaccharide or sugar alcohol, and additionally sprayed and dried with its surface coated with such saccharide (JP-A 61-134319), but it takes a long time to dry the coating sugar solution described above. Further, some of these saccharides are highly hygroscopic to cause various problems such as an unfavorable effect in preservation.

Further, there is proposed a method wherein a hardly dispersible food is mixed with an erythritol crystal having a particle size of 149 µm or less to improve the water dispersibility (JP-A 11-18698), but such fine powdered erythritol is liable to solidify and is difficult to handle, with no satisfied effect to improve the dispersibility. JP-A 11-123053 describes that a whey protein can be mixed with a sugar alcohol powder to give a product which is improved in the dispersing solubility, but with a insufficient effect to improve the dispersibility.

### BRIEF DESCRIPTION OF THE DRAWING

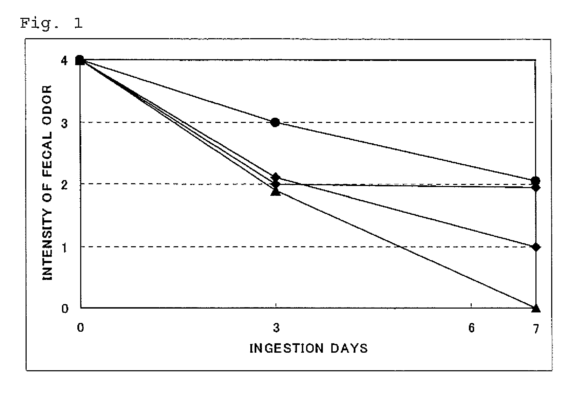

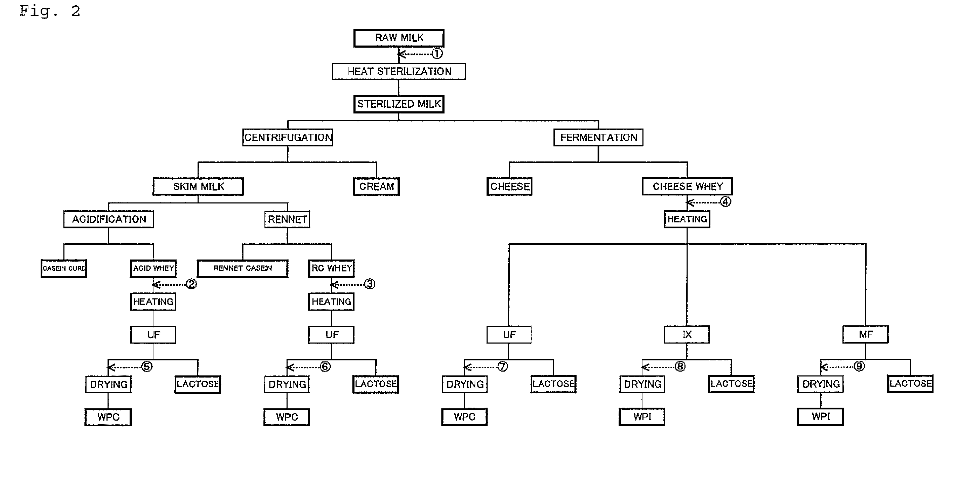

Fig. 1 is a graph showing sensory evaluation points by an individual in Effect Testing of Example 3-2. Fig. 2 is a flow chart representing the process for producing a whey protein with the time for adding glucide (from 1 to 9 (circled numbers in Fig. 2)) shown. WPC: whey protein concentrate, WPI: whey protein isolate, RC whey: Rennet casein whey, UF: ultrafiltration, IX: ion-exchange method, MF: microfiltration method.

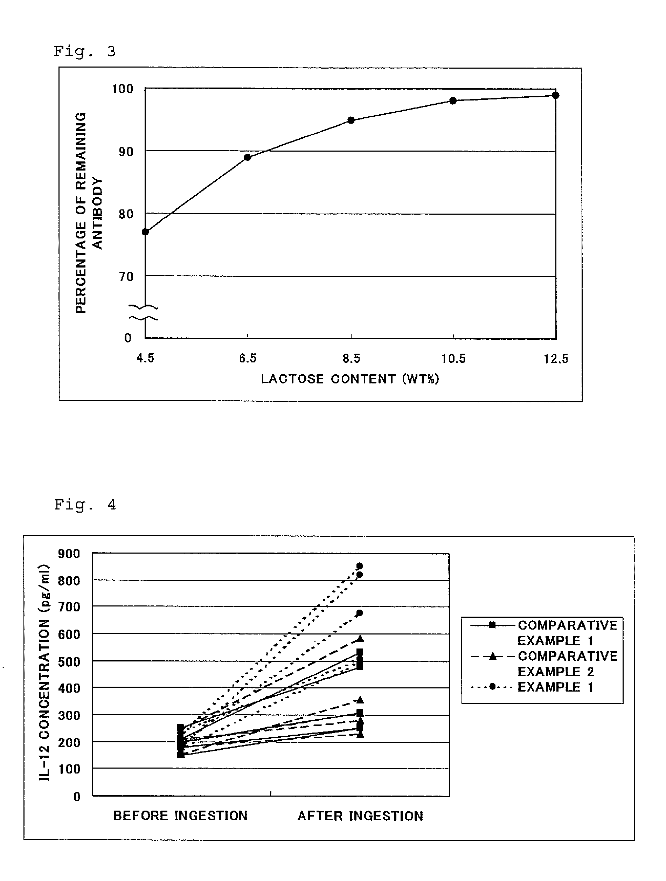

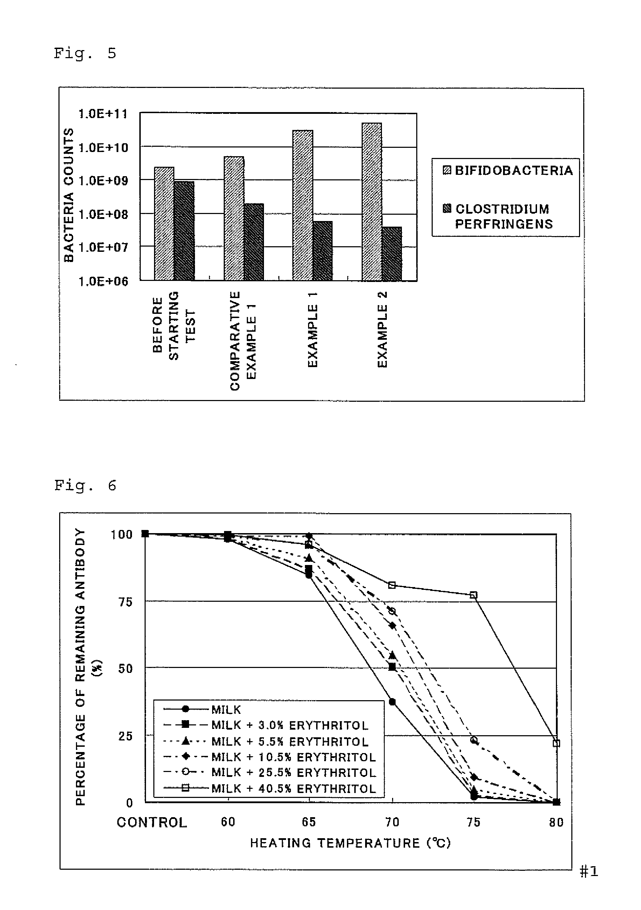

Fig. 3 is a graph showing a percentage of remaining antibody after raw milk was supplied with 0 to 8% by mass of lactose and heated for 30 minutes at 65°C. Fig. 4 is a graph showing effects of functional composition of the present invention on IL-12 production conducted in Examples 7-8.
Fig. 5 is a graph showing bifidobacteria and Clostridium perfringens counts in feces as measured in Example 10-1, Comparative Examples 10-1 and 10-2.

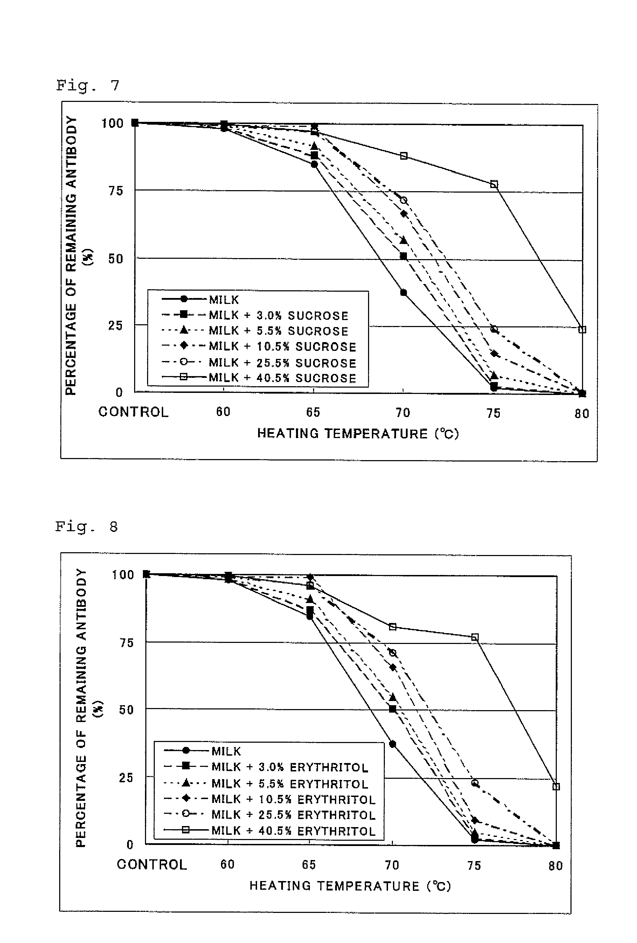

Fig. 6 is a graph showing the percentage of remaining antibody in various foods after they were supplied with whey protein and heated at 60 to 72°C for 30 minutes. Fig. 7 is a graph showing the percentage of remaining antibody after cow milk was supplied with whey protein and sucrose and heated at 60 to 80°C for 30 minutes. Fig. 8 is a graph showing the percentage of remaining antibody after cow milk was supplied with whey protein and erythritol and heated at 60 to 80°C for 30 minutes.

### Disclosure of the Invention

The present invention is a functional composition or food comprising whey protein, antibody derived from milk or antibody. The present invention includes the following aspect of the invention.

The present invention is a functional composition comprising an antibody and an immunostimulatory substance as an effective component (the invention aspect 7).

The present invention is a composition for treating rheumatoid arthritis comprising an antibody having antirheumatic effects as an effective component (the invention aspect 6).

The present invention is a method for producing an antibody-containing fermentation food, wherein raw milk from cow or the other mammal than cow, which is not heated at 63°C or more, is supplied with one or more of lactobacilli or bifidobacteria and fermented (the invention aspect 11) .

The present invention is an antibody-containing food comprising 1.5 mg/g or more of antibody and 10⁷/g or more of one or more bacteria selected from the group consisting of lactobacilli and bifidobacteria (the invention aspect 12).

The present invention is an antibody-containing apparent food comprising 1.5 mg/g or more of antibody derived from milk, with the proviso that it does not contain the lactobacilli and bifidobacteria by 10⁷/g or more (the invention aspect 13).

The present invention is a whey protein food comprising whey protein and glucide, cocoa powder, a powdery non-aggregating and insoluble matter, green tea, aloe, turmeric, pumpkin, red grape juice or tomato powder (the invention aspects 4, 9, 15, 1 and 2).

The present invention is a fecal odor-reducing agent comprising whey protein (the invention aspect 3).

The present invention is an excellently water-dispersible protein composition comprising a powdered or granulated protein derived from milk and a powdery non-aggregating and insoluble matter (the invention aspect 8) .

The present invention is a whey protein food comprising whey protein, an oligosaccharide and a water-soluble dietary fiber (the invention aspect 10).

The present invention is a method for producing whey protein, wherein a glucide is added at least at any time during the process for producing the whey protein to increase the percentage of remaining antibody (the invention aspect 14).

### Detailed Description of the Invention

The aspects of the present invention will be explained below. Materials disclosed in each aspect of the invention can also be used in other aspect of the invention.

### The Invention Aspect 6

An object of the present invention is to provide a composition having functions for preventing/treating and recurrence-preventing rheumatoid arthritis.

The present inventors made a strenuous study to solve the matters described above. As a result, it has been found that bovine, goat, sheep and chicken can be immunized with Type II collagen and killed cells of a Gram-negative enterobacteria alone or in their mixture as a vaccine to produce their respective antibodies, which bovine, goat, and sheep secrete in their respective milks, and chicken secretes in egg yolk, to incorporate in a food or a medicine, thereby to solve the problems. The finding completes the present invention. In other words, the food or the medicine comprising those antibodies can be ingested to react a Type II collagen derived from another food and endotoxin produced by bacteria in mouth or intestine with their respective antibodies, thereby inactivating them or preventing them from the absorption from gastrointestinal tract, and that the ingestion is useful for preventing rheumatoid arthritis or improving and treating the disease. The finding completes the present invention.

Namely, the present invention is the followings:
1) A composition for treating rheumatoid arthritis comprising an antibody having antirheumatic effect as an effective component.
2) The composition for treating rheumatoid arthritis according to 1) described above, wherein the antibody having antirheumatic effect is an anti-Type II collagen antibody.
3) The composition for treating rheumatoid arthritis according to claim 9, wherein the anti-Type II collagen antibody is at least one of anti-chicken Type II collagen antibody, anti-bovine Type II collagen antibody and anti-porcine Type II collagen antibody.
4) The composition for treating rheumatoid arthritis according to claim 8, wherein the antibody having antirheumatic effect is an anti-bacterial endotoxin antibody.
5) The composition for treating rheumatoid arthritis according to claim 8, wherein the antibody having antirheumatic effect is a mixture of an anti-Type II collagen antibody and an anti-bacterial endotoxin antibody at an appropriate ratio.
6) The composition for treating rheumatoid arthritis according to claim 8, comprising at least one of anti-Type II collagen antibody and anti-bacterial-endotoxin antibody by a total antibody amount of 0.1% by mass or more.

The present invention can provide a composition which contains an antibody and can be used for treating rheumatoid arthritis by inhibiting the induction of arthritis and preventing or treating rheumatoid arthritis, wherein the composition can be ingested as a food or a medicine to react the antibody with Type II collagen from another food or a toxin produced by bacteria in mouth or intestine.

According to the present invention, a domestic animal vaccinated with the above two antigens known as factors causing rheumatoid arthritis to produce their respective antibodies, which are then separated from milk, egg, and blood and incorporated in a food or a medicine. The invention will be explained below in reference with every item.

### 1. Antigens for use in immunization

In the present invention, as antigen, Type II collagens from animals and Gram-negative bacteria or the endotoxin thereof (LPS) are preferably used.
1) Commercial products (Chondrex) can be used for Type II collagens from bovine, swine and chicken. Type II collagens from chicken, bovine and swine may be used alone, and may be mixed at an optional combination to give a mixed vaccine which is efficient and economic.
2) Antigens for use in immunization to produce a bacterial endotoxin antibody include Gram-negative bacteria parasitic on a human body which belong to genera as shown below. Namely, they include Fusobacteria, Veillonella, Megasphaera, Neisseria, Moraxella, Branhamella, Acinetobacter, Citrobacter, Enterobacter, Escherichia, Hafnia, Klebsiella, Morganella, Proteus, Providencia, Salmonella, Serratia, Shigella, Yersinia, Vibrio, Aeromonas, Plesiomonas, Haemophillus, Pasteurella, Pseudomonas, Legionella, and are treated with formalin or heated to give their killed bacteria which are used as inactivated bacterial vaccines. They may be used alone or in their combination for a vaccine, and are preferable to use efficiently in a mixed vaccine. A common method described in books is useful in culturing bacteria.
3) An animal lives symbiotically with microorganisms, and has its inherent microorganisms in the gastrointestinal tract, with the microorganisms common to human. Those microorganisms become antigens, allowing the animal to produce antibodies to keep. They are called as natural antibodies. Antibody to endotoxin of Gram-negative bacteria common to human as well represents as a natural antibody, and can also be used.

### 2. Selection of animal

Usually, it has been known that an animal is injected with its autoantigen or a homogenous antigen to produce no antibody. Further, an animal is usually immunized with a larger heterogeneity of antigen to produce a larger amount of antibody. Therefore, for example, in order to produce an antibody to bovine Type II collagen, a chicken is desirably immunized with bovine Type II collagen to collect the antibody in the chicken egg yolk. Further, in order to produce an antibody to chicken Type II collagen, a cow can be immunized with chicken Type II collagen to get the antibody in the milk or serum. In this way, an animal is inoculated with a heterologous origin of Type II collagen. It is efficient and economic that a plurality of collagens from heterologous origins are mixed to inject as a mixed vaccine.

As bacterial components are antigenic to all animals, they can be mixed to inject as a mixed vaccine.

### 3. Method for inoculating with antigen

In a method for inoculating an animal such as milk cow, milk goat, sheep and chicken, any antigen may be used in an aqueous solution or suspension. A proteinous antigen such as collagen is desirably mixed with various immunization adjuvants to administer. For one immunization, 10 to 1000 µg of protein by dry mass is dissolved or suspended in 0.1 to 10 ml to prepare a water-in-oil emulsion using a same amount of, for example, Freund's complete and incomplete adjuvants, TiterMax Gold or Ribi Adjuvant R-730, and injected intradermally, subcutaneously, or intramuscularly at one to ten locations by 0.1 to 10 ml per one location. This will be conducted weekly to bimonthly.

To produce antibody to endotoxin, 0.1 to 10 ml of 0.01% to 10% by wet mass of one killed bacterial culture or a mixture of a plurality of killed bacterial cultures is administered subcutaneously, intramuscularly, subcutaneously or intravenously once in 1 to 4 weeks. Antibody to endotoxin can be produced by immunizing together with adjuvant. A water-in-oil emulsion is prepared using a killed bacterial culture with, for example, Freund' s complete and incomplete adjuvants, TiterMax Gold or Ribi Adjuvant R-730, and injected intradermally, subcutaneously, intramuscularly at one to different ten locations. This will be conducted weekly to bimonthly.

### 4. Collecting of antibody

1) Collecting of milk antibody
   Milk is collected by hand or using milking machine according to a common method.
   Because an antibody is heated over 72°C to lose the activity, it should be avoided from excessive heating until a final product. For sterilization, raw milk is appropriately treated with heating at 72°C or below, UV-ray irradiation sterilization or irradiation sterilization. Milk can be centrifuged to defat, and spray-dried to get a skim milk, or defatted, supplied with hydrochloric acid to adj ust the pH to 4.6, filtered to remove deposited casein, and ultrafiltered to remove low molecular components such as lactose and minerals to get a whey, or additionally ultrafiltered to remove lactalbumin and lactoglobulin, and spray-dried at a low temperature or freeze-dried to get a powdery antibody composition having an antibody content of 0.1% to about 90% with a main fraction of antibody protein contained. Usually, 1 ton of raw milk with removed colostrums provides 10 to 100 g, average 30 g, of antibody in a total amount. Further, a commercially available whey comprising antibody, whey protein concentrate (WPC), whey protein isolate (WPI) can also be used.
2) Collecting of serum antibody
   The blood of an immunized animal has a high antibody content, and thus may be an excellent material.
   Collected blood is centrifuged to get a serum. The serum is treated with a 1/3 saturated ammonium sulfate solution to precipitate a gamma-globulin fraction, which is then filtered to collect, dissolved in water, desalted, and spray-dried at a low temperature or freeze-dried to get a powder. Usually, The powder has generally an antibody content of 70 to 90%, and 1 kg of blood provides approximately 15 g of a total antibody.
3) Collecting of chicken egg antibody
   1 kg of chicken egg, which is collected from immunized chickens to determine using GIT kit (Cosmo Bio Co-, Ltd.), provides usually 0. 9 to 1.5 g of IgY antibody with a purity of 90%. The antibody solution is spray-dried at a low temperature or freeze-drying into a powder.

### 5. Method of determining antibody

In order to determine an antibody amount, two methods are used. One method measures a total amount of antibody regardless of the antigen specificity, and another method uses ELISA (Fujii K, et al. J Immunol Methods 1989; 124: 63-70) to measure the amounts of antibodies to their respective antigens.
1) Measuring a total amount of antibody
   Protein G column is commercially available (from Amersham Biosciences and others) as antibody purification affinity column for efficiently purifying antibody. The column can be passed through with an antibody-containing solution at a nearly neutral pH to adsorb almost 100% of antibody. The column is washed and passed through with an acidic solution having a pH of 2.5 to 2.8 to release the antibody from the column. The 1% solution of the antibody has an absorbance of approximately 14 at an ultraviolet wavelength of 280 nm, which is common regardless of animal kind, allowing determination of the amount of antibody from the absorbance.
2) Measurement of total IgY
   Total IgY can easily be determined using the commercially available adsorption column (HiTrap IgY Purification HP, Amersham Biosciences) for an egg yolk antibody. An antibody-containing solution is passed through the column with 20 mM phosphate buffer containing 0.5 M potassium sulfate at pH 7.5 to adsorb IgY antibody, which is then washed with the same solution, eluted with 20 mM phosphate buffer at pH 7.5 to collect, and determined from its absorbance.
3) Measurement of affinity purified specific antibody by ELISA

Collagen and bacterial endotoxin (LPS) are covalently linked to Actigel (Sterogene) or a CNBr activated sepharose (Amersham Bioscience) to use as an antigen affinity column for purifying antibody. An antibody solution to purify was applied to the column to adsorb only a target antibody while non-adsorbed substances such as immunoglobulin are washed away with a neutral buffer. The antibody bound onto the column was eluted with 0.1M glycine buffer at pH 2.8. The eluted antibody was further applied to protein G column to get a purified antibody, which is then used as an authentic sample to prepare a standard curve by ELISA (enzyme immunoassay) for determination. Condition for sensitizing wells with each antigen and blocking solutions are mentioned below.

### Measuring anti-collagen antibody by ELISA:

Collagen was diluted with 0.15 M potassium phosphate buffer, pH 7.2, to get a 10 µg/ml solution, 50 µl of which was put into an ELISA plate well to coat at 4°C through day and night. For blocking, a rabbit serum heated at 56°C for 30 minutes is supplied with 0.05 M Tris and 0.15M sodium chloride to get a pH 7.8 adjusted solution, which is used as a blocking solution. This blocking solution is also used for diluting an analyte to determine the antibody.

### ELISA for measuring bacterial endotoxin antibody:

LPS, which is extracted from Gram-negative bacteria with a trichloroacetic acid Solution, is added in 0.05 M carbonate buffer at pH 9.5 to prepare a 5 µg/ml solution, with which the ELISA plate wells are then coated. The wells are blocked with a phosphate-buffered saline containing 0.1% bovine serum albumin.

Anti-bovine antibody, anti-goat antibody, anti-sheep antibody and anti-IgY antibody, which are labeled with peroxidase respectively, are reacted according to the published documents and colored with OPD as a substrate to measure the absorbance, which is used to determine the total amount of antibody or the amount of specific antibody based on a standard curve made using the authentic sample of the antibody.

### 6. Preparing antibody-containing composition

A preparation containing the antibody to Gram-negative bacterial endotoxin and a preparation containing the antibody obtained by immunizing with Type II collagen can be used alone or in their combination to prepare an antibody-containing composition. As required, it can be supplied with the other edible materials and excipients than the antibody-containing preparations to form a food or a medicine. The final product has preferably a total antibody content of 1% or more.

### The Invention Aspect 7

An object of the present invention is to provide a functional composition having stronger immunomodulatory effect and stronger intestine-regulating effect.

The present inventors made a strenuous study to find that an antibody is mixed with an immunostimulatory substance to ingest simultaneously, in other words, a substance for passive immunity is combined with a stimulatory substance for active immunity to give stronger immunomodulatory and intestine-regulating effect than these respective substances alone, thus achieving the present invention.

Therefore, the present invention is the following:
1) A functional composition comprising an antibody and an immunostimulatory substance as the effective components.
2) The functional composition according to claim 8, wherein the antibody is an antibody derived from milk.
3) The functional composition according to claim 8 or 9, wherein the immunostimulatory substance is a bacteral body or components of a bacterium, excluding lactobacilli and bifidobacteria, which produces no toxin.
4) The functional composition according to claim 1 or 2, wherein the immunostimulatory substance is a fungus body or components of a mold/yeast.
5) The functional composition according to claim 1 or 2, wherein the immunostimulatory substance is a mushroom or components of a mushroom.
6) The functional composition according to claim 1 or 2, wherein the immunostimulatory substance is an enzymatic degradation product of materials selected from bacteria, molds/yeasts and mushrooms.
7) The functional composition according to claim 1 or 2, wherein the immunostimulatory substance is SOD (Superoxide dismutase).

According to the present invention, an antibody is mixed with an immunostimulatory substance to ingest simultaneously, resulting in stronger immunomodulatory and intestine-regulating effect. The immunostimulatory substance activates active immunity in a gastrointestinal tract, meanwhile the antibody serves for passive immunity there, so that simultaneous ingestion of both of them brings about strong immunomodulatory and intestine-regulating effects.

Specifically, ingestion of antibody, namely, passive immunity in a gastrointestinal tract decreases bad bacteria, improves intestinal environment, and further allows a simultaneously ingested immunostimulatory substance to function easily, providing higher immunostimulatory effect than the ingested immunostimulatory substance alone. Thus, Th1/Th2 balance of immune system further shifts toward Th1 dominance, thereby to activate macrophages, natural killer cells (NK cells) and killer T cells (CTL cells) , resulting in the enhanced production of interleukin 12 (IL-12) and interferon γ (IFN-γ), and thus immune function in the whole body is improved.

In the present invention, as the antibody, antibodies derived from egg, serum and the like can also be used, and an easily available antibody derived from milk is preferably used. Whey protein, which has a high concentration of antibody derived from milk, can be used as the antibody in the present invention. As whey protein, usual commercially available ones such as whey protein concentrate (WPC), whey protein isolate (WPI) and desalted whey powder can be used, and ones with a higher antibody content are preferred. For separating whey protein from whey, various methods are adopted, and any method, such as ultrafiltration, micro-filtration, cross-flow micro-filtration, and ion-exchange can be used.

Milk as a material for whey protein may be milk from any mammal, for example, cow, goat, sheep, horse and buffalo, and further the milk may be collected from a vaccinated mammal or an unvaccinated mammal.

In the present invention, the immunostimulatory substance for use in combination with an antibody includes all known immunostimulatory substances, and preferably, bacteria, excluding lactobacilli and bifidobacteria, which produce no toxin, molds/yeasts, the fungus bodies or fungal components of mushrooms, the enzymatic degradation products of materials selected from bacteria, molds/yeasts and mushrooms and SOD.

As the bacterium, excluding lactobacilli and bifidobacteria, which produces no toxin, any bacterium can be used if it is a bacterium excluding lactobacilli and bifidobacteria and produces no toxin. For example, Bacillus natto and amino acid-producing bacteria (genera of Corynebacterium, Brevibacterium, Macrobacterium) can be used. These bacteria may be used as viable cells or as killed bacteria, or the dried product and the extract of an effective component, and the dried product thereof can be used.

As the mold/yeast, any mold/yeast can be used if it is edible. For example, beer, bread, torula, black, and marine yeasts, Aspergillus oryzae, and Rhizopus oligosporus can be mentioned. Their dried products and extracts, or dried products of the extracts, and further, culture media of these yeasts, extracts of culture media, dried products thereof can be used.

As the mushroom, any mushroom can be used if it is edible. For example,Agaricus,Meshimakobu, Yamabusitake, Suehirotake, Kawaratake, Houkitake, Hanabiratake, Sanagitake, Naratake, Matutake, Maitake, Himematutake, plant worm, Mannentake (Ganoderma lucidum), Ganoderma amboinense, Kohukisarunokosikake, Kabanoanatake (Fuscoporia obliqua), Siitake, Enokidake, Nameko, Honsimeji, Kikurage, Sirokikurage, Eringi, Kurokawa may be mentioned. The dried products and extracts of fungus bodies comprising fruit bodies and mycelia of these mushrooms or dried products of the extracts and the like can be used.

The enzymatic degradation product of bacteria, molds/yeasts and mushrooms described above can also be used. As the enzyme, any enzyme may be used, and a glycosidase enzyme such as lysozyme and an endopeptidase proteinase such as bromelain can be preferably used in their combination.

Further, as the SOD, any SOD, for example, plant-, animal- and microorganism-derived SOD can be used. Particularly, as the SOD, a prolamin-treated SOD (US-B6045809) is preferably used because it is orally ingested to show stronger immunostimulatory effect than the untreated SOD.

In addition, as the other immunostimulatory substance, proteinases such as glutathione, lysozyme and bromelain; lactoferrin, fucoidan, casein degradation products, vitamins A, C and E, B group vitamins, carotenoid, minerals (iron, zinc, selenium), coenzyme Q10, α-lipoic acid, propolis, royal jelly, taurine, glucosamine, job's tear extract, Lo Han Kuo extract, glycyrrhizin, cat's claw, Echinacea, Serenoa repens, pycnogenol, catechin, polyphenol such as grapeseed extract, chlorella, algae such as Spirulina, and β-glucan derived grains (such as barley, oat and job's tear) can be used. Their commercially available products can be used.

As the immunostimulatory substance, a phenol compound and polyphenol of grapeseed extract, phenol compound and polyphenol derived from olive can also be used.

In the present invention, as the functional material, aspartic acid, arginine, glycine, glutamine, γ-amanobutyric acid, choline, theanine, phosphatidylserine, Ubiquinone, Rutin, lutein, astaxanthin, vitamins D and K, eicosapentaenoic acid (EPA), docosahexaenoic acid (DHA), octacosanol, gluconic acid, gingko leaf extract, olive, hop extract, soybean isoflavone, n-3, n-6 andn-9 unsaturated fatty acids, kale, young leaves of barley and the like may be added.

In the present invention, there is no limitation in a method for mixing the antibody and the immunostimulatory substance and also in the form of the composition.

The functional composition preferably comprises 10 mg/g or more of the antibody, and more preferably from 10 mg/g to 300 mg/g. Further, the functional composition preferably comprises 1% by mass or more of the immunostimulatory substance, and more preferably from 1% by mass to 95% by mass.

### The Invention Aspect 11

An object of the present invention is to provide a food which can keep its original good taste of raw milk and, at the same time, can kill hazardous bacteria, such as food poisoning bacteria (such as E. coli and Staphylococcus aureus) and pathogens (such as tuberculosis bacteria), and can be ingested to keep the activity of an antibody contained in raw milk, and a production method thereof.

The present inventors made a strenuous study to solve the matters described above. As a result, it has been found that raw milk is supplied with one or more of lactobacilli or bifidobacteria to ferment, allowing to kill hazardous bacteria without heat sterilization needed, to maintain its original good taste as raw milk, and further to exempt the contained antibody from losing the activity if the raw milk is not heated at 63°C or higher, though it was beyond expectation. This finding provides a novel antibody-containing fermented food, and leads to the present invention.

Namely, the present invention is the following:
1) A method for producing an antibody-containing fermented food, wherein raw milk from a cow or the other mammal than the cow is supplied with one or more of lactobacilli or bifidobacteria to ferment without heating at 63°C or more.
2) The method for producing an antibody-containing fermented food according to 1) described above, wherein the raw milk is previously supplied with an organic acid to adjust the pH to 5.8 or less before it is supplied with one or more of lactobacilli or bifidobacteria.
3) The method for producing an antibody-containing fermented food according to 1.) or 2) described above, wherein the raw milk is defatted to ferment.
4) An antibody-containing fermented food obtained by the production method according to any one of 1) to 3) described above.

According to the invention described in claim 14, there is provided a method wherein raw milk from a cow or the other mammal than the cow is supplied with lactobacilli or the like to ferment, allowing to kill the contained hazardous bacteria such as E. coli, Staphylococcus aureus and pathogens (such as tuberculosis bacteria), and to produce a fermented food which enables the contained and ingested antibody to display its function and maintains the original good taste as raw milk. According to the invention described in claim 15, raw milk is supplied with an organic acid to adjust the pH to 5.8 or less and fermented, allowing inhibition of hazardous bacteria from the growth and creation of a suitable environment for the growth of lactobacilli and the like. Further, according to the invention described in claim 16, a defatted raw milk can be used to provide a fermented low fat food.

In the present invention, raw milk is referred to milk which is collected from a cow or the other mammal than the cow followed by being exempted from heating at 63°C or higher, and is preferably used within 48 hours after collecting. In order to produce yogurt which is a typical lactobacilli fermented food using cow milk as a material, the raw milk is heated, for example, by ultra high temperature sterilization to previously kill hazardous bacteria such as a usually contaminating saprophytic bacteria, E. coli, Staphylococcus aureus and the other food poisoning bacteria which can pollute the raw milk. But, in the present invention, raw milk is fermented without heating at 63°C or higher, because the raw milk is heated at the temperature to inactivate an antibody, to lose its own good flavor, and to release unpleasant heat-caused odor, thereby to break the balance of original tastes of raw milk. Even if raw milk is contaminated with hazardous bacteria such as E. coli lactobacilli and the like added is fermented to inhibit the bacteria from growth and kill them during fermentation, causing none of risk such as food poisoning. Further, in the present invention, raw milk is not heated at 63°C or higher, causing no inactivation of an antibody contained therein and enabling the antibody to exert sufficiently the function for health.

Preferably, raw mil is previously supplied with an organic acid to adjust the pH to 5.8 or less, and further with lactobacilli to ferment. The pH adjusted to 5.8 or less can inhibit hazardous bacteria from growth, thereby further decreasing a risk such as food poisoning. As the useful organic acid, for example, lactic, citric, malic, succinic, tartaric, adipic, acetic, gluconic, fumaric and phosphoric acids can be mentioned, and among them, lactic acid is preferred.

Further, defatted raw milk can be used to give a low fat fermented food, which is suitable for a diabetic patient who needs low-calorie food and a person trying to lower the body mass. As the method for removing a fat component, a usual method for separating milk fat content such as centrifugation may be used.

In the present invention, as the lactobacillus to add, Lb. delbrueckii subsp. bulgaricus, Lb. delbrueckii subsp. lactis, Lb. delbrueckii subsp. delbrueckii, Lb. acidphilus, Lb. casei, Lb. reuteri, Lb. brevis, Lb. gasseri, Lb. johnsonii, Lb. arabinosus and Lb. helveticus which belong to Lactobacilli; Stc. thermophilus belonging to Streptococci; Lc. lactis subsp. lactis, Lc. lactis subsp. cremoris, Lc. plantarum and Lc. raffinolactis in Lactococci; P. halophilus and P. pentosaceus in Pediococci; Leu. mesenteroides and Leu. lactis in Leuconostocs; and Ec. faecalis and Ec. faecium in Enterococci can be mentioned.

As the bifidobacterium, B. bifidus, B. longum, B. breve, B. infantis, and B. adolescentis in Bifidobacteria can be mentioned.

Further, yeast can be added for complex fermentation as conducted in so called kefir yogurt for example.

Fermentation is conducted under usual fermentation conditions, in other words, fermentation and cultivation is conducted at from 20 to 50°C for 8 to 24 hours.

Raw milk can be supplied with auxiliary materials to add usually in a food, including a sweetener such as sugar and a flavor, to ferment, unless the materials affect the fermentation.

It has been known that lactobacilli and bifidobacteria have effects such as intestine regulating action, blood cholesterol lowering action, blood pressure lowering action, anticancer and immunostimulatory effects (Science and Technology of Lactobacillus, Japan Scientific Academy Press) . The fermented food obtained by the present invention, which gives a combination of the function for health by lactobacilli or bifidobacteria and that by antibody, is a quite useful food expected to give a synergistic effect. Further, the food provides the raw milk's original good taste, because it is exempted from heating at a high temperature.

The fermented food obtained as described above can be eaten as it is like a yogurt. Further, it can be used as a food material, for example, for yogurt mousse, yogurt cake, frozen yogurt or yogurt sauce.

Further, the antibody-containing fermented food obtained by the production method of the present invention can be supplied with auxiliary materials to add usually to a food, including a sweetener such as sugar and a flavor to produce a lactobacillus beverage, a bifidobacterium beverage and a sour beverage. Further, the antibody-containing fermented food can be dried into powder, and thus provides a dried food such as a powdered milk and a skim milk containing an antibody.

### The Invention Aspect 12

The problem of the present invention is to provide a food which comprises an antibody at a higher concentration and gives a combination of the function possessed by the antibody and that by lactobacilli or bifidobacteria.

The present inventors have found that an edible material such as whey protein, which contains an antibody at a higher concentration, can be incorporated and further combined with lactobacilli or bifidobacteria to solve the problem described above, and thus achieved the present invention.

Therefore, the present invention is the following:
1) An antibody-containing food comprising 1.5 mg/g or more of an antibody and 10⁷/g or more of one or more bacteria selected from the group consisting of lactobacilli and bifidobacteria.
2) The antibody-containing food according to claim 18, comprising 3 mg/g or more of an antibody.
3) The antibody-containing food according to claim 18 or 19, wherein the antibody is an antibody present in whey protein.
4) The antibody-containing food according to any one of claims 18 to 20, wherein the antibody is a colostral antibody.
5) The antibody-containing food according to any one of claims 18 to 21, further comprising the other component having an immunostimulatory function.

According to the present invention, a food, which contains an antibody at a high concentration together with lactobacilli or bifidobacteria, can enhance simultaneously passive immunity and active immunity, wherein the former is acquired by ingesting the antibody from outside a body and the latter is activated inside the body by the lactobacilli or the bifidobacteria, thereby providing a synergistic effect in immunostimulation. One or more of lactobacilli and bifidobacteria are contained at 10⁷/g or more, allowing inhibition of the other saprophytic bacteria, in particular food poisoning bacteria. According to the invention described in claim 19, a food comprising an antibody at a higher concentration can be provided. Further, according to the invention described in claim 20, a food comprising an antibody present in whey protein at a high concentration can be provided. According to the invention described in claim 21, a food having a high antibody content which comprises an antibody present in colostrum at a high concentration can be provided. Further, according to the invention described in claim 22, a food, which is further supplied with the other component having an immunostimulatory function than a component described above to give a more excellent immunostimulatory function, can be provided.

The antibody-containing food of the present invention preferably comprises an antibody which is contained in an antibody source material having the antibody at a high concentration, such as whey protein and colostrum. Because the antibody concentrations of whey protein and colostrum are high, they are suitable for an antibody source material for a food comprising an antibody at a high concentration. Antibody contained in raw milk is directly transferred in some commercially available whey protein to be present therein at a high concentration without its activity denatured or inactivated. A commercially available whey protein can be used, but its antibody may be denatured at a high temperature. Therefore it is preferred to use the whey protein which has been rarely heated in the production process, for example, those produced by an ion-exchange membrane method or an ultrafiltration membrane method. More preferably, the whey protein comprising active antibody at 1% or higher is more preferable.

Material milk for whey protein may be from any mammal such as cow and goat. Further, the milk may be any milk collected from a vaccinated mammal and an unvaccinated mammal.

If colostrum is used as an antibody source material, it may be any mammalian colostrum such as cow and goat, and further, the colostrum may be any collected from vaccinated mammal and an unvaccinated mammal. Colostrum means milk secreted within 4 or 5 days after secretion starts. It has been known that colostrum contains antibody at a higher concentration by dozens to hundreds times than usual milk (Minoru Ohta, Chemistry and Biology, 37 (2), 107-112 (1999)).

Whey protein, colostrum and an edible material containing them may be heated to sterilize in such a degree that they are lowered in antibody content. They should not be heated at a temperature of 76°C or more . Further, they may be non-thermally sterilized, for example, by filtration, electron beam, gamma ray, ultraviolet ray, high-voltage pulse, oscillating magnetic field, flash pulse, ultrasound, or high pressure.

The antibody-containing food of the present invention comprises the antibody at 1.5 mg or more per 1 g of the food, and preferably at 1.5 to 500 mg/g. Further, a food, which has an antibody content of 3 mg/g or more, is more preferable because it is expected to show the immunostimulatory function of the antibody and its synergistic effect with lactobacilli and bifidobacteria. Further, preferable content is from 3 to 500 mg/g.

In the present invention, lactobacillus in the invention aspect 11 can be used. Further, the other lactobacilli can also be used.

Bifidobacteria in the invention aspect 11 can be used. The other bifidobacteria can also be used.

The lactobacilli and the bifidobacteria may be bacteriocin-producing strains or not. In addition, it has been proved that not only viable cells but also dead bacteria of lactobacilli and bifidobacteria have immunostimulatory effects, thus either of which can be added.

Further, the antibody-containing food of the present invention comprises one or more bacteria selected from the group consisting of lactobacilli and bifidobacteria at 10⁷ or more per 1 g of the food. A food, which contains these lactobacilli or/and bifidobacteria at 10⁷/g or more, is expected to give an effect such as immunostimulatory effect. The preferable content is from 10⁷ to 10¹²/g.

The antibody-containing food of the present invention, which comprises any one or more bacteria from the group consisting of lactobacilli and bifidobacteria, is not particularly limited in the form to supply the bacteria, and may be a fermented food and a non-fermented food, wherein the former can be obtained by supplying the bacteria to milk such as cow milk and soy milk to ferment, and the latter can be obtained by mixing a bacterial culture medium or dried bacterial culture in the main materials. Further, the component of the antibody-containing food of the present invention can be used as the principal component.

The antibody-containing food of the present invention can further comprise an antibody and the other components having an immunostimulatory function than lactobacilli and bifidobacteria (hereinafter, the other immunostimulatory components) . The food can contain the other immunostimulatory components to enhance further immunostimulatory function. As the other immunostimulatory components, for example, following components with known immunostimulatory function can be mentioned: basidiomycetes such as Agaricus, Meshimakobu and Ganoderma; DNA, nucleic acid composition such as nucleotide; propolis; chitin; chitosan; lactoferrin; fucoidan; Echinacea; Paud' arco; rice bran; yeast; SOD-like substances such as vitamins C and E, β-Carotene and polyphenol ; SOD; and Sekirenkaca.

Further, the present invention may be supplied with functional materials described in the invention aspect 7 described above.

If the antibody-containing food of the present invention is a fermented food, there is no limit in the production method and a usual production method can be used. In that case, preferably, the fermented culture is heated to sterilize and then supplied with an antibody source material such as whey protein and colostrum. After thermal sterilization, the antibody source material may be added before, after or during fermentation.

A fermentation medium is supplied with whey protein, colostrum or the like, and then usually supplied with a fermentation starter such as lactobacilli to ferment and culture at 20 to 50°C for 8 to 24 hours. Further, yeast can be added for complex fermentation as found in so called kefir yogurt.

The non-fermented food can be produced by simply adding whey protein or colostrum, and a bacterial culture solution or a dried bacterial culture to the other materials of an antibody-comprising food. There is no limit in method for adding bacteria. Non-fermented food herein means a food which lactobacilli or/and bifidobacteria are proliferated separately from the other materials and then added in the other materials in a non-fermentation manner to produce. The non-fermentation manner includes cooling of the bacterial culture and drying of the bacterial cells

In the present invention, the fermented food includes, in addition to yogurt, yogurt mousse, yogurt cake, frozen yogurt, dried fermented milk, and yogurt sauce. Further, the non-fermented food includes various types of food such as processed milk, ice cream, dessert, soft drink, tablet, and powdery food.

Further, the antibody-containing food of the present invention can comprise auxiliary materials usually to add in food, for example, sweetener such as sugar, flavor and polysaccharide thickening agent.

### The Invention Aspect 13

The subject of the present invention is to provide an apparent food offering an enjoyable taste, which comprises an antibody at a higher concentration, and a production method thereof.

The present inventors have found that a processed food is supplied with an edible material such as whey protein which contains an antibody at a higher concentration under a particular condition to solve the problem described above, and that the product thus obtained can be heated under a certain condition to exempt the antibody from inactivation with the activity remained by 50% or more, thus achieve the present invention.

Therefore, the present invention is the following:
1) An antibody-containing apparent food comprising an antibody derived from milk at 1.5 mg/g or more (with the proviso that it does not comprise lactobacilli and bifidobacteria at 10⁷/g or more).
2) The antibody-containing apparent food according to 1) described above, comprising the antibody derived from milk at 3 mg/g or more.
3) The antibody-containing apparent food according to 1) described above, comprising the antibody derived from milk at 10 mg/g or more.
4) The antibody-containing apparent food according to any one of 1) to 3) described above, wherein the antibody derived from milk is an antibody contained in whey protein.
5) The antibody-containing apparent food according to any one of 1) to 4) described above, further comprising the other component having an immunostimulatory function than the antibody derived from milk.
6) The antibody-containing apparent food according to any one of 1) to 5) described above, wherein the antibody-containing apparent food is a dairy product or frozen dessert
7) A method for producing the antibody-containing apparent food according to any one of 1) to 6) described above, wherein the apparent food comprising an antibody derived from milk or a portion of the apparent food comprising an antibody derived from milk is heated to sterilize under such a condition that 50% or more of the antibody may remain.
8) The method for producing the antibody-containing apparent food according to 7) described above, wherein the portion of the apparent food comprising an antibody is adjusted to have a glucide content of 7.5% by mass or more and then heated to sterilize.

According to the invention described in claim 23, a food, which comprises an antibody at a high concentration to have an expected immunostimulatory effect, can be provided. According to the invention described in claim 24, a food which comprises an antibody at a higher concentration can be provided. Further, according to the invention described in claim 25, a food which comprises an antibody at an extremely high concentration can be provided. According to the invention described in claim 26, a food which comprises an antibody contained in whey protein at a high concentration can be provided. Further, according to the invention described in claim 27, a food, which further comprises the other component having an immunostimulatory function than an antibody derived from milk to show a more excellent immunostimulatory function, can be provided. As shown in the invention described in claim 28, an antibody-containing apparent food can offer both good taste as a dairy product or frozen dessert and immunostimulatory effect. According to the invention described in claim 29, a food which is heated with 50% of the antibody remaining can be produced. According to the invention described in claim 30, an apparent food, which comprises a glucide at 7.5% by mass to increase the stability of an antibody, can be produced to have a high amount of the remaining antibody.

The antibody-containing food of the present invention preferably comprises an antibody which is contained in an antibody source material having the antibody at a high concentration, such as whey protein and colostrum. Because the antibody concentrations of whey protein and colostrum are high, they are suitable for an antibody source material for a food comprising an antibody at a high concentration. Antibody contained in raw milk is directly transferred in some commercially available whey protein to be present therein at a high concentration without its activity denatured or inactivated. A commercially available whey protein can be used, but its antibody may be denatured at a high temperature. Therefore it is preferred to use the whey protein which has been rarely heated in the production process, for example, those produced by an ion-exchange membrane method or an ultrafiltration membrane method. More preferably, the whey protein comprising active antibody at 1% by mass or higher is more preferable.

Material milk for whey protein may be from any mammal such as cow and goat. Further, the milk may be any milk collected from a vaccinated mammal and an unvaccinated mammal.

If colostrum is used as an antibody source material, it may be any mammalian colostrum such as cow and goat, and further, the colostrum may be any collected from vaccinated mammal and an unvaccinated mammal. Colostrum means milk secreted within 4 or 5 days after secretion starts. It has been known that colostrum contains antibody at a higher concentration by dozens to hundreds times than usual milk (Minoru Ohta, Chemistry and Biology, 37 (2), 107-112 (1999)).

The antibody-containing apparent food of the present invention comprises the antibody derived from milk at 1.5 mg or more per 1 g of the food, and preferably at 1.5 to 200 mg/g. Further, a food, which has an antibody content of 3 mg/g or more, is more preferable because it is expected to show the immunostimulatory function of the antibody. Further, preferable content is from 3 to 200 mg/g. The food, which has an antibody content of 10 mg/g or more, is more preferred, because it is expected to show a further high immunostimulatory function of the antibody. Even more preferably, the content is 10 to 200 mg/g.

The antibody-containing food of the present invention can further comprise the other components having an immunostimulatory function than the antibody. The food can contain the other immunostimulatory components to enhance further immunostimulatory function. As the other immunostimulatory component than the antibody, for example, following components with known immunostimulatory function can be mentioned: basidiomycetes such as Shiitake, Enokidake, Agaricus, Meshimakobu and Ganoderma; DNAs of prokaryotic and eukaryotic cells and nucleic acid compositions such as nucleotide; propolis; chitin; chitosan; lactoferrin; fucoidan; Echinacea; Paud'arco; rice bran; yeast; SOD-like substances such as vitamins C and E, carotenoid, green tea component and polyphenol; pharmaceutical compositions comprising SOD (US-B6045809); and Chinese medicine components such as Sekirenka, Korean ginseng and turmeric. The component is not limited to these, and any component can be included if it has an immunostimulatory function. They can also be used as an extract, its dried powder or the like.

Further, the functional material described in the invention aspect 7 may be added to the present invention.

In the present invention, the apparent food indicates a common name of "the product apparently acknowledged as food", which can easily be recognized under common sense as "food" from its appearance, shape and components, and the term is described in "Reality of Advertisement of Drugs, Cosmetics etc. '94" (Yakugyo Jiho Co., Ltd.) edited by Pharmaceutical Division in Tokyo Metropolitan Government Bureau of Public Health in Inspection and Guidance Division in Pharmaceutical Affairs Bureau. It is a food or drink which does not have a form such as tablet, capsule and powder usually used in pharmaceuticals and dietary supplements, and includes processed food, cooked food, confectioneries, beverage, and seasonings.

The apparent food of the present invention includes various forms of food, for example, dairy products (wherein the content of lactobacilli and bifidobacteria is less than 10⁷/g) such as processed milks and milk beverages; palatable beverages such as fruit juice beverage, vegetable juice beverage, soft drinks and soy milk; seasonings such as dressings and sprinkles; frozen desserts such as ice and soft creams; desserts such as jellies; margarines, spreads, topping sauces, condensed milk, whipped cream, coffee creams; confectioneries such as chocolates and chewing gums.

The antibody-containing apparent food of the present invention can be obtained by a usual production method, and there is no limit in the production method. Whey protein or colostrum, which is one of antibody source materials, can be added to produce or cook the antibody-containing apparent food. After the addition of the antibody source material, the product is preferably heated to sterilize under such a condition that 50% or more of the antibody may remain, and then subjected to cook or production. As the condition for heating to sterilize with 50% or more of the antibody remaining, for example, heating at 60°C for 30 minutes or more moderate may be mentioned, but, as shown in Fig. 6, the temperature can further be increased depending on food. A glucide is contained at 7.5% by mass or more to stabilize the antibody against heat, allowing heating at a higher temperature. Thus, the glucide can be added to heat to sterilize.

Further, an antibody-containing apparent food can also be obtained by following way. The antibody-containing portion such as whey protein and colostrum is heated to sterilize under such a condition that 50% or more of the antibody may remain, while the other portion of an edible material with no antibody contained is heated to sterilize under a usual sterilization condition, and then the both portions are aseptically mixed.

Further, an antibody-containing apparent food can also be obtained using a nonthermal sterilization such as filtration, electron beam, gamma ray, ultraviolet ray, high-voltage pulse, oscillating magnetic field, flash pulse, ultrasound, high pressure treatment and DPCO₂ (Dense Phase Carbon Dioxide).

Fig. 6 shows the rate of remaining antibody in each food after the food is supplied with whey protein and heated at various temperatures for 30 minutes. As the food, a commercially available cow milk, potage soup and vegetable juice beverage were chosen respectively, and supplied with 2% by mass, 3% by mass and 3% by mass of whey protein to prepare their respective samples. In addition, an aqueous 10% by mass whey protein solution was prepared. Then the samples were heated at a temperature of 60 to 72°C to measure the amount of the antibody in each food, and their rates of the remaining antibody were calculated. The method for measuring the amount of antibody will be represented in Examples described below. In Fig. 6, ●: 2% by mass whey protein was added to cow milk (pH 6.5) , ■: 3% by mass whey protein was added to potage soup (pH 5.3), ▲: 3 % by mass whey protein was added to vegetable juice beverage (pH 4.5), □: 10 % by mass aqueous solution of whey protein (pH 6.4).

As shown in Fig. 6, with cow milk (pH 6.5 after adding whey protein), 50% or more antibody remained after heating at 68°C for 30 minutes; with aqueous 10% by mass whey protein solution (pH 6.4 as above), approximately 50% of it remained after heating at 67°C for 30 minutes; and with potage soup (pH 5.3 as above), 50% of it remained after heating at 66°C for 30 minutes. Meanwhile, with vegetable juice beverage (pH 4.5 as above), the remaining rate was 50% or more after heating at 60°C for 30 minutes. In this way, it can be seen that 50% or more of antibody can be left by selecting a thermal sterilization condition for each subject food. From the facts above, a heating condition can be determined depending on food by preliminary test to allow 50% or more of antibody to remain, and thus the food may be heated to sterilize under the determined condition or more moderate.

Meanwhile, the inventors tried to search a substance which can enhance heat stability of antibody and discovered that the object can be achieved by the addition of glucide.

Fig. 7 shows the rates of remaining antibody after a commercially available cow milk was supplied with whey protein and sucrose and then heated at various temperatures for 30 minutes. In this test, 0% by mass and 3% by mass, 5.5% by mass, 10. 5% by mass, 25.5% by mass and 40.5% by mass sucrose were added. Cow milk contains originally 4.5% by mass glucide, and thus test samples contained the glucide 4.5% by mass, 7.5% by mass, 10% by mass, 15% by mass, 30% by mass and 45% by mass, respectively. The test samples were supplied with 2% by mass of whey protein and heated at a temperature of 60 to 80°C to measure the antibody amount of the cow milk, and the rates of remaining antibodies were calculated. In Fig. 7, ●: 2% by mass of whey protein was added to cow milk (glucide content 4.5% by mass), ■: 3.0% by mass of sucrose and 2% by mass of whey protein were added to cow milk (glucide content 4.5% by mass) , ▲: 5. 5% by mass sucrose and 2% by mass whey protein were added to cow milk (glucide content 4.5% by mass), ◆:10.5% by mass sucrose and 2% by mass whey protein were added to cow milk (glucide content 4.5% by mass), o:25.5% by mass sucrose and 2% by mass whey protein were added to cow milk (glucide content 4.5% by mass) , □: 40.5% by mass sucrose and 2% by mass whey protein were added to cow milk (glucide content 4.5% by mass).

Fig. 8 shows the results of a test which was conducted in the same way as in the test described above, but erythritol was used in place of sucrose. In addition, fructose and galacto-oligosaccharide were used to give a similar heat stability. In Fig. 8, ●: 2% by mass whey protein was added to cow milk (glucide content 4.5% by mass) , ■: 3.0% by mass erythritol and 2% by mass whey protein were added to cow milk (glucide content 4.5% by mass) , ▲: 5.5% by mass erythritol and 2% by mass whey protein were added to cow milk (glucide content 4.5% by mass), ◆: 10.5% by mass erythritol and 2% by mass whey protein were added to cow milk (glucide content 4.5% by mass), o: 25.5% by mass erythritol and 2% by mass whey protein were added to cow milk (glucide content 4.5% by mass), □: 40.5% by mass erythritol and 2% by mass whey protein were added to cow milk (glucide content 4.5% by mass).

As shown in Figs 7 and 8, the sample, which had a glucide content of 7.5% by mass, was heated at 70°C for 30 minutes with 50% or more of the antibody remaining in any case. Further, the glucide content is increased to increase the heat stability of antibody. The sample, which had a glucide content of 45% by mass, was heated at 77°C for 30 minutes with 50% or more of the antibody remaining.

From the facts above, it can be seen that an antibody-containing apparent food can be heated at a higher temperature, because the antibody-containing portion which is adjusted to have a glucide content of 7.5 % by mass or more to increase the stability of the antibody against heat, and that the apparent food can be heated at a same temperature with the higher rate of remaining antibody than a food treated otherwise.

As the glucide having these effects, monosaccharides such as glucose and fructose; disaccharides such as sucrose and lactose; oligosaccharides such as fructo-oligosaccharide and isomalt-oligosaccharide; glycerin; sugar alcohols such as sorbitol and erythritol can be used. The glucides may be added alone or in combination of them, and glucide originally present in a food material can also be used.

Further, the apparent food can contain materials usually added in food such as sweetener, acidulant, flavor and thickening polysaccharide.

### The Invention Aspect 4

The subject of the present invention is to provide a whey protein food which the young to the elderly can ingest by reducing milk smell, puckery, acrid and astringent tastes possessed by whey protein to improve flavor, and is improved in water absorption and dispersibility so that it may be mixed with other foods to give "floating masses" that can be disentangled in a short time, namely, a food which is easy to mix and eat.

The present inventors made a strenuous study to solve the problem above. As a result, it has been found that whey protein can be supplied with a glucide such as oligosaccharide to reduce milk smell, puckery, acrid and astringent tastes possessed by whey protein, and to improve its water absorption so that it may be mixed with water, a liquid food such as cow mil and tomato juice, or a pasty food such as yogurt to give "floating masses" that can be disentangled in a short time, thereby achieving the present invention.

Therefore, the present invention is the following:
1) A whey protein food comprising 100 parts by mass of whey protein and 5 to 1000 parts by mass of glucide.
2) The whey protein food according to 1) described above, wherein the glucide is one or more indigestible saccharides.
3) The whey protein food according to 2) described above, wherein the indigestible saccharide is selected from fructo-oligosaccharide, lactosucrose, lactulose, isomalt-oligosaccharide and galacto-oligosaccharide with no α-galactosyl bond.
4) The whey protein food according to any one of 1) to 3) described above, comprising having a granular form.
5) The whey protein food according to any one of 1) to 4) described above, wherein the whey protein food has an antibody content of 5 mg/g or more.
6) The whey protein food according to any one of 1) to 5) described above, wherein the whey protein food has an antibody content of 10 mg/g or more.
7) The whey protein food according to any one of 1) to 5) described above, wherein the whey protein food has an antibody content of 50 mg/g or more.

According to the invention described in claim 32, whey protein can be mixed with a glucide to reduce milk smell, puckery, acrid and astringent tastes possessed by the whey protein, to improve its flavor, further to make it tastier, and to improve its water absorption for mixing with a liquid or pasty food. According to the invention described in claim 33, whey protein can be mixed an indigestible saccharide as the glucide to give expected synergistic effect of the immunostimulatory function possessed by the antibody and the physiological function possessed by the indigestible saccharide. According to the invention described in claim 34, the whey protein food has markedly improved flavor. According to the invention described in claim 36, the glucide-containing whey protein food can be granulated to improve greatly the water absorption for mixing with a liquid or pasty food. According to the invention described in claim 37, the whey protein food can be adjusted to have an antibody content of 5 mg/g or more to give sufficiently the immunostimulatory function possessed by the antibody. According to the invention described in claim 38, the whey protein food can be adjusted to have an antibody content of 10 mg/g or more to give more sufficiently the immunostimulatory function possessed by the antibody. According to the invention described in claim 39, the whey protein food can be adjusted to have an antibody content of 50 mg/g or more to give even more sufficiently the immunostimulatory function possessed by the antibody.

Usually, a whey protein solution is separated (fractionated) from whey such as cheese whey and acid whey and then dried to produce whey protein. But whey protein fractionated from any whey may be used in the present invention. Further, there is no limit in the method for fractionating whey protein from whey. Besides a method for crystallizing lactose to remove, any other method, for example, micro-filtration, cross flow microfiltration, ion-exchange and ultrafiltration can also be used. Milk for the material whey, either of any mammalian milk such as cow, goat, sheep, horse and buffalo, milk collected from a vaccinated mammal or an unvaccinated mammal may be served.

As whey protein, a commercially available whey protein can be used. These whey proteins include whey protein concentrate (WPC), whey protein isolate (WPI), desalted whey powder.

Antibody present in raw milk remains in whey protein. Thus, the whey protein can be dried to give a powder which contains concentrated antibody to use as a good supplier of antibody.

As the glucide for use in the present invention, besides the indigestible saccharide described below, any glucide useful for food such as glucose, fructose, sucrose, isomerized sugar and dextrin may be used. These glucides can be used alone or in combination of two or more. The whey protein can contain a glucide to reduce milk smell and puckery, acrid and astringent tastes possessed by whey protein, to improve its water absorption for mixing with a liquid or pasty food to improve the dispersibility.

As the indigestible saccharide, any indigestible saccharide usable for food can be used. For example, oligosaccharides such as fructo-oligosaccharide, lactosucrose, lactulose, isomalt-oligosaccharide, soybean-oligosaccharide, galacto-oligosaccharide, soybean-oligosaccharide, xylo-oligosaccahraide, raffinose, palatinose-oligosaccharide, nigero-oligosaccharide, gentio-oligosaccharide, trehalose, the coupling sugar, pectin-oligo and cyclodextrin; sugar alcohols such as sorbitol, maltitol, erythritol, reduced palatinose, lactitol, mannitol and xylitol; inulin; indigestible dextrin; and resistant starch can be mentioned. As the glucide, a commercially available usual product can be used. In the present invention, the indigestible saccharide means a saccharide hardly digested with a digestive enzyme inside the body, so that a highly digestible saccharide coated with a lipid-soluble substance or promilans such as zein and gliadin can be used as an indigestible saccharide herein.

Indigestible saccharide becomes a growth factor for intestinal bifidobacteria, and those bifidobacteria affect immune system in gastrointestinal tract, thereby activating active immunity. Meanwhile, whey protein, which contains an antibody for activating passive immunity, is ingested with an indigestible saccharide to activate both active and passive immunities, thus to give expected strong immunostimulatory effect. Further, because some indigestible saccharides have such functions as intestine-regulating effect, mineral absorption facilitating effect, anti-caries effect, blood cholesterol inhibiting effect, and liver function improving effect, the whey protein can be mixed with the indigestible saccharide to give expected synergistic effects for these effects and effects for improving flavor and water absorption of whey protein and immunostimulative effect.

Among the indigestible saccharides, fructo-oligosaccharide, lactosucrose, lactulose, isomalt-oligosaccharide and galacto-oligosaccharide with no α-galactosyl bond contained are excellent in improving flavor and preferred. In particular, galacto-oligosaccharide with no α-galactosyl bond contained exhibits more flavor-improving effects, even if used in a small amount, than the other oligosaccharide.

In the whey protein food of the present invention, the saccharide is preferably from 5 to 1000 parts by mass based on 100 parts by mass of whey protein. More preferably, it is from 5 to 900 parts by mass. If saccharide content is less than 5 parts by mass, the effects for decreasing milk smell, puckery, and astringent tastes possessed by whey protein and effect for improving water absorption are insufficient. Meanwhile, if the content exceeds 1000 parts by mass, the content of whey protein becomes lower, resulting in the decreased antibody content in the whey protein food, thus the object of ingesting whey protein is difficult to achieve.

The whey protein food of the present invention can be produced by adding a glucide to a powdery whey protein to mix. Further, it can be produced by a method for adding the glucide described above to a whey protein solution fractionated from whey to dry into a powder. There is no limit in method for drying the whey protein solution supplied with glucide and, for example, a method such as spray-drying and freeze-drying can be employed.

The whey protein food of the present invention can be granulated to improve the water absorption of whey protein, facilitating to mix with other foods. Whey protein, which is poor in water absorption, is added into water, cow milk and the like to give easily a "floating masses". However, whey protein food contains a glucide such as oligosaccharide described above to improve the water absorption, and further granulated to improve further the water absorption and dispersibility, facilitating to mix with water or the other food such as cow milk, tomato juice and yogurt with no "floating masses" generated.

The granulation can be conducted by a common method such as spray granulation and extrusion granulation. It is believed that glucide such as oligosaccharide described above serves as a binder in granulation, but CMC (carboxymethyl cellulose) and the like may be added as a binder. The granulation is desirably conducted under a condition without the product temperature risen over 70°C. If the product temperature exceeds 70°C, antibody present in whey protein can be inactivated. Unless the product temperature exceeds 70°C, appropriate conditions for throughput, blower temperature, blower volume and the like may be set.

The whey protein food of the present invention, which comprises 5 mg/g or more of an antibody derived from milk, can preferably give the expected immunostimulatory function of the antibody. Further, the whey protein food of the present invention, which comprises 10 mg/g or more of an antibody derived from milk, can more preferably give the expected immunostimulatory function of the antibody. The whey protein food comprising 50 mg/g or more is particularly preferred. The whey protein has preferably an antibody content of 5 to 500 mg/g, more preferably 10 to 500 mg/g, and further more preferably 50 to 500 mg/g in.

Further, the whey protein food of the present invention can comprise food additive materials usually to add in a food such as high-sweet-level sweetener, acidulant, flavor, emulsifier and polysaccharide thickening agent.

The whey protein food of the present invention, which is treated to be free from milk smell and puckery, acrid and astringent tastes possessed by whey protein is improved in its flavor, can be ingested tastier and easier. Further, the whey food is added to the other foods to offer a good water absorption, comparing to a powder comprising whey protein alone. Further, the whey food is granulated to improve further the water absorption, thereby to allow mixing with a liquid or pasty food. Further, the whey food of the present invention, which contains an indigestible glucide, becomes a growth factor for intestinal bifidobacteria and has physiological functions such as hardly-carious, low-calorie and intestine-regulating effects, thus providing these physiological functions.

### The Invention Aspect 9

The aspect of the invention 9 comprises the following in addition to 1) to 4) in 4.
5) The whey protein food according to any one of 1) to 3) described above, wherein the whey protein is granular and the glucide is powder.
6) The whey protein food according 1) to 5) described above, wherein the glucide is 30 parts by mass or more based on 100 parts by mass of the whey protein.
7) The whey protein food according to any one of 1) to 6) described above, wherein the content of antibody derived from milk is 5 mg/g or more.
8) The whey protein food according to any one of 1) to 7) described above, wherein the content of antibody derived from milk is 10 mg/g or more.
9) The whey protein food according to any one of 1) to 8) described above, wherein the content of antibody derived from milk is 50 mg/g or more.

According to the invention described in claim 40, whey protein, which is granulated and supplied with a powder glucide, further improves the water absorption, thereby to easily disperse in cow milk and the like in which powders are difficult to disperse. According to the invention described in claim 41, a more dispersibile and readily soluble whey protein food can be obtained. According to the invention described in claim 37, by setting the content of antibody derived from milk 5 mg/g or more, the immunostimulatory function possessed by antibody can be obtained sufficiently. According to the invention described in claim 38, by setting the content of antibody derived from milk 10 mg/g or more, the immunostimulatory function possessed by antibody can be obtained more sufficiently. According to the invention described in claim 39, by setting the content of antibody derived from milk 50 mg/g or more, the immunostimulatory function possessed by antibody can be obtained even more sufficiently.

In the present invention, for whey protein and glucide, the milk protein and the glucide in the invention aspect 4 can be used. Dextrins with various DE (Dextrose Equivalent) values can be commercially available, any of which can be used, and cyclodextrin and cluster-dextrin can also be used.

As the indigestible saccharide, in addition to those in aspect 4, alginic acid, polydextrose, arabinoxylan, pullulan, curdlan and the like can be mentioned.

The similar amount of saccharidea and the method for mixing saccharide as in the invention aspect 4 can be used.

The whey protein food of the present invention can be prepared by a method wherein whey protein is granulated and supplied with aforementioned powdery glucide. This improves the water absorption of the whey protein food, and facilitates to mix with the other foods. Particularly, this allows the food to mix rapidly with cow milk in which whey protein powder is hardly dispersible. The granule is not limited in size, and usually the mean granule size is preferred to be around 0. 02 to 2 mm. The whey protein food, which contains the glucide at 30 parts by mass or more based on 100 parts by mass of the granulated whey protein, is preferably excellent in water absorption. As the glucide increases, the properties of granulated whey protein are impaired, so that the glucide is appropriately contained at 300 parts by mass or less.

Antibody derived from milk and other additives can be used in the same manner as in the invention aspect 4. The Invention Aspect 15

The present inventors have found that whey protein is supplied with one or more oligosaccharides selected from fructo-oligosaccharide, lactosucrose, lactulose, isomalt-oligosaccharide and galacto-oligosaccharide with no α-galactosyl bond contained, to reduce milk smell and puckery and astringent tastes possessed by whey protein, to improve its water absorption, so that the whey protein is mixed with a liquid food such as cow milk or a pasty food such as yogurt to give "floating masses" which are disentangled in a short time, thus achieving the present invention.

Therefore, the present invention is the following:
1) A whey protein food comprising whey protein and from 5 to 900 parts by mass of one or more oligosaccharides selected from fructo-oligosaccharide, lactosucrose, lactulose, isomalt-oligosaccharide and galacto-oligosaccharide with no α-galactosyl bong contained based on 100 parts by mass of the whey protein.
2) The whey protein food according to claim 35, having a granular form.

According to the present invention, whey protein is supplied with a defined amount of one or more oligosaccharides selected from fructo-oligosaccharide, lactosucrose, lactulose, isomalt-oligosaccharide and galacto-oligosaccharide with no α-galactosyl bond contained, to reduce milk smell and puckery and astringent tastes possessed by whey protein, to improve flavor, further to make whey protein tastier, to improve its water absorption, and to facilitate mixing with a liquid or pasty food. The whey protein can be granulated to improve the water absorption greatly, providing an easier mixing with a liquid or pasty food and an easier tasting.

In the present invention, usual whey protein described above can be used. For a separation method from raw milk, any method such as micro-filtration, cross-flow micro-filtration and ion-exchange can also be used.

Milk as a material for preparing whey protein for use in the present invention may be derived from any mammal such as cow, goat, sheep and horse. Further, the milk may either be milk collected from a vaccinated mammal or an unvaccinated mammal.

Forfructo-oligosaccharide, lactosucrose, lactulose, isomalt-oligosaccharide and galacto-oligosaccharide with no α-galactosyl bond contained for use in the present invention, a commercially available usual product can be used. In particular, a galacto-oligosaccharide with no α-galactosyl bond contained, even if used in a small amount, exhibits more flavor-improving effect than the other oligosaccharide.

The whey protein food of the present invention comprises from 5 to 900 parts by mass of these oligosaccharide based on 100 parts by mass of the whey protein. If oligosaccharide content is less than 5 parts by mass, the effect for decreasing milk smell and puckery and astringent tastes possessed by whey protein and the effect for improving the water absorption are insufficient. Meanwhile, if it exceeds 900 parts by mass, the content of whey protein becomes so low that the object of ingesting whey protein will be difficult to achieve.

Further, the whey protein food of the present invention is granulated to improve further the water absorption, facilitating to mix with the other foods. Whey protein alone is added in water, cow milk and the like to generate a "floating mass" because of its poor water absorption. However, the whey protein food comprising the oligosaccharide described above has an improved water absorption. Further, the whey protein food, which is granulated and added to the other food such as cow milk and yogurt, is improved in water absorption to give no "floating mass" and facilitated to mix.

It is believed that the oligosaccharide described above serves as a binder in granulation, however, CMC (carboxymethyl cellulose), glucide and the like may further be added as a binder. Further, the granulation can be conducted by a common method such as spray granulation and extrusion granulation.

The present whey protein food can comprise the materials in the aspect 4 usually added in food.

### The Invention Aspect 3

The subject of the present invention is to provide a fecal odor-reducing agent with high deodorant effect.

The present inventors have examined the function of whey protein from various view points and found that this whey protein has effects of significantly improving fecal odor, thus achieving the present invention. The further inspection has showed that its main effective component is an antibody contained in whey protein.

Therefore, the present invention is the following:
1) A fecal odor-reducing agent comprising whey protein.
2) The fecal odor-reducing agent according to 1) described above, wherein the antibody in the whey protein is an effective component,
3) The fecal odor-reducing agent according to claim 42 or 43, wherein the content of the antibody is 0.5% by mass or more.
4) The fecal odor-reducing agent according to any one of claims 42 to 44, further comprising an indigestible saccharide.

According to the present invention, an antibody contained in whey protein is used as an effective component to provide an excellent fecal odor-reducing agent. The agent, which has an antibody content of 0.5% by mass or more, can give a markedly improving effect. Further, the agent, which is supplied with an indigestible saccharide, can give an even higher improving effect.

In the present invention, for whey protein, commercially available usual products such as whey protein concentrate (WPC), whey protein isolate (WPI) and desalted whey powder can be used. A whey protein with a higher antibody content is preferred. As a method for separating whey protein from whey, any method such as micro-filtration, cross-flow micro-filtration and ion-exchange can also be used.

Milk as a material for preparing whey protein for use in the present invention may be derived from any mammal such as cow, goat, sheep, horse and buffalo. Further, the milk may either be collected from a vaccinated mammal or an unvaccinated mammal.

Usually, whey protein has been used in improving the physical properties of ham, sausage, and the like, and improving qualities of bread, yogurt, and the like. Recently, because of its amino acid sequence similar to that of human milk, its high nutritive value, its inclusion of all essential amino acids, its extremely high absorption rate, and further its high content of branched amino acids, it has been used in products for mainly athletes and people with a high exercise tolerance as dietary supplements to supply protein.

Some whey protein contains a remaining antibody derived from raw milk of material. The fecal odor-improving effect of the present invention is an effect obtained from such antibody contained in whey protein. Therefore, the fecal odor-reducing agent of the present invention preferably comprises 0.5% by mass or more of an antibody from whey protein for improving fecal odor. Further, in the adult, ingestion of 50 mg or more of an antibody from whey protein per day is effective for improving fecal odor.

Further, the fecal odor-reducing agent in the present invention contains an indigestible saccharide to increase its effect more significantly. As the indigestible saccharide, if it is an indigestible saccharide useful for food, any saccharide can be used. For example, oligosaccharides such as fructo-oligosaccharide, lactosucrose, lactulose, isomalt-oligosaccharide, soybean-oligosaccharide, galacto-oligosaccharide, soybean-oligosaccharide, xylo-oligosaccahraide, raffinose, palatinose-oligosaccharide, nigero-oligosaccharide, gentio- oligosaccharide, trehalose, the coupling sugar, pectin-oligo and cyclodextrin; sugar alcohol such as sorbitol, maltitol, erythritol, reduced palatinose, lactitol, mannitol and xylitol; inulin; indigestible dextrin; resistant starch can be mentioned. For these glucides, commercially available usual products can be used. In the present invention, indigestible saccharide means a saccharide which is hardly digested with a digestive enzyme inside the body, so that a highly digestible saccharide coated with a lipid-soluble substance or promilans such as zein and gliadin can be used as an indigestible saccharide herein. 5 to 1000 parts by mass of the indigestible saccharide is preferably added based on 100 parts by mass of the whey protein.

Indigestible saccharide is a growth factor for intestinal bifidobacteria and has intestine-regulating effect too. Simultaneous ingestion of whey protein and indigestible saccharide provides their synergistic and more significant effect for improving fecal odor.

The fecal odor-reducing agent of the present invention can comprise, beisides whey protein as an effective component, food additive materials usually to add in food such as excipient, sweetener, acidulant, flavor, emulsifier, polysaccharide thickening agent and coloring agent.

In the present invention, there is no limit in the form of fecal odor-reducing agent. For example, it may be of tablet, powder, granule and capsule and also paste and liquid. Unless there is a heat-processing step which inactivates antibody after being added to whey protein, it can be in the form of a processed food.

### The Invention Aspect 1

The subject of the present invention is to provide a delicious-looking whey protein food, wherein milk smell and puckery and astringent tastes possessed by the whey protein are so much reduced to improve the flavor that the young to the elderly can ingest the food, and its water absorption is so much improved that the food can be mixed with the other food to give a "floating mass" which can be disentangled in a short time.

The present inventors have screened various food materials to solve the problem described above and, as a result, found that whey protein is supplied with cocoa powder to reduce milk smell and puckery and astringent tastes possessed by whey protein, and to improve its water absorption so that it can be mixed in water or a liquid food such as cow milk and soy milk to give a "floating mass" which is disentangled in a short time, and that the cocoa powder can give the food a delicious-looking color and a pleasant feeling, thus achieving the present invention.

Therefore, the present invention is the following:
1) A whey protein/cocoa food comprising whey protein and 1 to 10, 000 parts by mass of cocoa powder based on 100 parts by mass of the whey protein.
2) The whey protein/cocoa food according to claim 46, having a granular form.

According to the present invention, whey protein is supplied with a defined amount of cocoa powder to reduce milk smell and puckery and astringent tastes possessed by whey protein and to improve its flavor, and further to improve its water absorption so that the food can be mixed with water or a liquid food such as cow milk and soy milk to give a floating mass which is easily disentangled. Further, brown color of cocoa powder visually attracts an ingesting person, induces appetite and offers fun and a change. The food can be granulated to improve the water absorption greatly, providing an easier mixing with other liquid food and an easier ingestion.

In the present invention, for whey protein, commercially available usual products and those described in the invention aspects 4, 9, and 15 can be used.

Milk as a material for preparing whey protein for use in the present invention may be derived from any mammal such as cow, goat, sheep and horse. Further, the milk may either be collected from a vaccinated mammal or an unvaccinated mammal.

In the present invention, as the cocoa powder, usual one can be used. Cocoa powder is produced by, for example, screening cacao beans with a cleaner, crushing the beans with a separator, removing shells, reacting with an alkalinizing agent put in a reactor, roasting by a roaster, grinding by a grinder, milling the obtained cacao mass to obtain oil by a cocoa press, separating a portion of cocoa butter out, and grinding by a cocoa mill into a fine powder. However, there is no limit in the production method. Now, the cocoa powder is rich in functional components such as polyphenol and dietary fiber, therefore it is an excellent functional food. A cocoa powder with a high content of these functional components can also be used.

Whey protein is supplied with cocoa powder to reduce the bitterness of cocoa powder erases milk smell and puckery and astringent tastes possessed by whey protein, and the flavor and brownish color of cocoa powder make the whey protein/cocoa food feeling tastier. Further, the food contains cocoa powder to improve the water absorption, so that the food can be mixed well with a liquid food.

The whey protein/cocoa food of the present invention comprises 1 to 10, 000 parts by mass of cocoa powder based on 100 parts by mass of whey protein. If the cocoa powder content is less than 1 parts by mass, it gives insufficient effects for reducing milk smell and puckery and astringent tastes possessed by whey protein and for improving the water absorption, and gives the insufficient coloring. Meanwhile, if the cocoa powder content exceeds 10, 000 parts by mass, the content of whey protein is lowered, losing the meaning of ingesting whey protein.

Further, the whey protein-and-cocoa food of the present invention is granulated to improve further the water absorption, allowing to mix well with a liquid food. Whey protein alone is added in water, cow milk, soy milk and the like to generate a "floating mass" because of its poor water absorption. However, a whey protein/cocoa food comprising cocoa powder has an improved water absorption, and is further granulated to increase the water absorption more, and thus it is added to the other foods to generate no "floating mass" to mix well.

It is believed that cocoa powder contains glucide and fiber, so these component serve as a binder in granulation, however CMC (carboxymethyl cellulose), glucide and the like may be added as a binder. Further, the granulation can be conducted by a common method such as spray granulation and extrusion granulation.

The whey protein/cocoa food of the present invention can comprise materials usually added in food such as dairy products such as powdered skim milk, powdered whole milk and cream powder; sweeteners such as sucrose, lactose, erythritol and Stevia extract; acidulants such as citric acid and malic acid, sodium chloride, calcium carbonate, flavor, polysaccharide thickening agent.

As stated above, whey protein/cocoa food of the present invention erases milk smell and puckery and astringent tastes possessed by whey protein, making it tastier and easier to ingest. Further, it is added to the other food to offer good water absorption, compared with a powder comprising whey protein alone. Further, it is colored with cocoa powder to offer a tasty appearance. Further, it is granulated to improve the water absorption, allowing to mix well with a liquid food.

### The Invention Aspect 2

The subject of the present invention is to provide a delicious-looking whey protein food, wherein milk smell and puckery and astringent tastes possessed by whey protein is so much reduced to improve its flavor that the young to the elderly can ingest the food, and further its water absorption is so much improved that the food can be mixed with the other foods to generate a "floating mass" which is disentangled in a short time.

The present inventors have screened various food materials to solve the problem described above and, as a result, found that whey protein is supplied with one or more powders selected from cranberry, raspberry, blueberry and strawberry powders to reduce milk smell and puckery and astringent tastes possessed by whey protein, and to improve so much its water absorption that the food can be mixed with a liquid food such as cow milk or a pasty food such as yogurt to give a "floating mass" which is disentangled in a short time, and that the food is colored with a pigment derived from a natural material to provide a tasty and funsome appearance, thus achieving the present invention.

Therefore, the present invention is the following:
1) A whey protein food comprising whey protein and 1 to 900 parts by mass of one or more powders selected from cranberry, raspberry, blueberry and strawberry powders based on 100 parts by mass of the whey protein.
2) The whey protein food according to claim 48, having a granular form.

According to the present invention, the whey protein food is supplied with a defined amount of one or more powders selected from cranberry, raspberry, blueberry and strawberry powders to reduce milk smell and puckery and astringent tastes possessed by whey protein, and to improve its flavor, and to improve so much its water absorption that the food can be mixed with a liquid or pasty food to give a floating mass which is easily disentangled. Further, red to purple color of these powders visually attracts an ingesting person, induces appetite, and offers fun and a change. The food is granulated to improve the water absorption greatly, providing an easier mixing with the other liquid or pasty foods and an easier tasting.

In the present invention, for whey protein, commercially available usual products and those described in the invention aspects 4, 9, and 15 can be used.

Milk as the material for preparing whey protein for use in the present invention can be used in the same manner as in the invention aspect 1 described above.

In the present invention, as cranberry powder, a commercially available usual product can be used. Whey protein is supplied with cranberry powder to erase milk smell and puckery and astringent tastes possessed by whey protein, and to make the whey protein food feeling tastier. Further, the whey protein food is supplied with cranberry powder to improve the water absorption, allowing an easy mixing with a liquid or pasty food.

In the present invention, as raspberry powder, a commercially available usual product can be used. Whey protein is supplied with raspberry powder to erase milk smell and puckery and astringent tastes possessed by whey protein, and to make the whey protein food feeling tastier. Further, the whey protein food is supplied with raspberry powder to improve the water absorption, allowing an easy mixing with a liquid or pasty food.

In the present invention, as blueberry powder, a commercially available usual product can be used. Whey protein is supplied with blueberry powder to erase milk smell and puckery and astringent tastes possessed by whey protein, and to make the whey protein food feeling tastier. Further, the whey protein food is supplied with blueberry powder to improve the water absorption, allowing an easy mixing with a liquid or pasty food.

In the present invention, as strawberry powder, a commercially available usual product can be used. Whey protein is supplied with strawberry powder to erase milk smell and puckery and astringent tastes possessed by whey protein, and to make the whey protein food feeling tastier. Further, the whey protein food is supplied with strawberry powder to improve the water absorption, allowing an easy mixing with a liquid or pasty food.

The whey protein food of the present invention comprises 1 to 900 parts by mass of one or more powders selected from cranberry, raspberry, blueberry and strawberry powders based on 100 parts by mass of the whey protein. If the content of powder described above is less than 1 parts by mass, it gives insufficient effects for reducing milk smell and puckery and astringent tastes possessed by whey protein and for improving the water absorption, and provides the food with insufficient coloring. Meanwhile, if it exceeds 900 parts by mass, the content of whey protein is so much lowered that the object of ingesting whey protein will be difficult to achieve.

Further, the whey protein food of the present invention is preferably granulated, as described in the invention 4.

It is believed that the powders described above contain glucide, starch and fiber derived from their respective plants, and these components serves as a binder in granulation, however CMC (carboxymethyl cellulose), glucide and the like may be added as a binder. Further, the granulation can be conducted by a common method such as spray granulation and extrusion granulation.

The whey protein food of the present invention can also comprise materials usually added in food such as sweeteners such as sucrose, erythritol and Stevia extract; acidulants such as citric acid and malic acid, flavor and polysaccharide thickening agent.

As stated above, the whey protein food of the present invention erases milk smell and puckery and astringent tastes possessed by whey protein, to be tastier and more ingestible. Further, when it is added to other food, it offers good water absorption, compared with a powder comprising whey protein alone. Further, it is colored with a natural material to offer tasty appearance. Further, it is granulated to improve the water absorption, allowing easier mixing with a liquid or pasty food.

### The Invention Aspect 8

The subject of the present invention is to provide a protein derived from milk which is simply improved in dispersibility in water.

The present inventors made a strenuous study to solve the problem described above and, as a result, found that a powdered or granulated protein derived from milk is mixed with a powdery unsolidifiable and insoluble substance to improve the water dispersibility of the protein, and that the product thus obtained is further mixed with an indigestible saccharide to improve further the dispersibility, thus achieving the present invention.

Therefore, the present invention is the following:
1) A protein composition excellent in water dispersibility comprising a powdered or granulated protein derived from milk and a powdery unsolidifiable and insoluble substance.
2) The protein composition according to 1) described above, wherein the powdery unsolidifiable and insoluble substance is cellulose.
3) The protein composition according to 1) or 2) described above, wherein the powdered or granulated protein derived from milk is whey protein.
4) The protein composition according to any one of 1) to 3) described above, further comprising an indigestible saccharide.

According to the present invention, a powdered or granulated protein derived from milk can easily be dispersed in water, juice, coffee and the like, and further can be easily mixed to disperse even with a pasty food such as yogurt. Further, many powdery unsolidifiable and insoluble subjects have effects as dietary fibers, allowing to give expected intestine-regulating and constipation-improving effects. The product thus obtained contains further an indigestible saccharide not only to improve the water dispersibility, but also to give expected functions such as intestine-regulating effect, mineral absorption facilitating effect, anti-caries effect, effect for inhibiting the increase of blood cholesterol and effect for improving liver function.

The powdery unsolidifiable and insoluble substance for use in the present invention includes, besides plant-derived ones such as okara, wheat bran, defatted soy, and cellulose; chitosan, silica gel, calcium carbonate, magnesium carbonate, but is not limited to these. In particular, celluloses such as microcrystaline cellulose such as a commercially available Avicel (supplied by Asahi Kasei Corporation) and powder cellulose such as KC FLOCK (supplied by Nippon Paper Chemicals Co., Ltd.) are used to give high effects.

The powdered or granulated protein derived from milk for use in the present invention is not limited particularly, and includes, for example, powdered skim milk, full fat powdered milk, whey powder, milk protein, whey protein, casein. In particular, whey protein, which has an amino acid sequence similar to that of human milk, a high nutritive value, all essential amino acids, an extremely high absorption rate, and further a high content of branched amino acids, is more preferred. As the whey protein, a commercially available whey protein can be used, for example, whey protein concentrate (WPC), whey protein isolate (WPI), and desalted whey powder can be mentioned.

The protein composition contains preferably 1 to 50% by mass of powdery unsolidifiable and insoluble substance. The content of less than 1% by mass gives the insufficient effect for improving dispersibility, and the content exceeding 50% by mass decreases relatively the protein content to lose its value as powder protein.

The protein composition of the present invention can further contain an indigestible saccharide to improve further the dispersibility in water. Any saccharide, if it is indigestible saccharide useful for food, can be used. For example, oligosaccharides such as fructo-oligosaccharide, lactosucrose, lactulose, isomalt-oligosaccharide, soybean-oligosaccharide, galacto-oligosaccharide, soybean-oligosaccharide, xylo-oligosaccahraide, raffinose, palatinose-oligosaccharide, nigero-oligosaccharide, gentio- oligosaccharide, trehalose, the coupling sugar, pectin and cyclodextrin ; sugar alcohols such as sorbitol, maltitol, erythritol, reduced palatinose, lactitol, mannitol and xylitol; inulin; indigestible dextrin; resistant starch can be mentioned. For these glucides, commercially available usual products can be used. In the present invention, the indigestible saccharide means a saccharide which is hardly digested with a digestive enzyme inside the body, so that a highly digestible saccharide coated with a promilans such as zein and gliadin or hardened oil can be used as the indigestible saccharide herein.

The protein composition described above can be supplied with an indigestible saccharide not only to improve the dispersibility, but also to give expected functions such as intestine-regulating effect, mineral absorption facilitating effect, anti-caries effect, effect for inhibiting the increase of blood cholesterol and effect for improving liver function. Further, whey protein, which is selected as a powdered or granulated protein derived from milk, contains an antibody to activate passive immunity. Meanwhile an indigestible saccharide is a growth factor for intestinal bifidobacteria, which affect immune system in gastrointestinal tract to activate active immunity. Whey protein is ingested together with an indigestible saccharide to activate both passive immunity and active immunity, thereby to give expected strong immunostimulatory effect.

Further, the protein composition of the present invention can comprise food materials and food additive materials usually to add in food, such as a high-sweet-level sweetener, acidulant, flavor, emulsifier, and polysaccharide thickening agent.

As a method for mixing powder protein with a powdery unsolidifiable and insoluble substance, indigestible saccharide and the like, common methods for use in mixing powder, for example, mixing methods using mixers such as V-shaped, horizontally cylindrical type, vertical screw type or dual conic type mixer may be mentioned.

The protein composition of the present invention may be a powder composition, and can be granulated to improve further the dispersibility. Powder protein is previously formed into a protein granule, which is then mixed with a powdery unsolidifiable and insoluble substance, an indigestible saccharide and the like. Alternatively, powder protein may be mixed with a powdery unsolidifiable and insoluble substance, an indigestible saccharide and the like, and then granulated. Further, powder protein is mixed with a powdery unsolidifiable and insoluble substance, an indigestible saccharide and the like, then granulated, and further mixed with powder protein, a powdery unsolidifiable and insoluble substance, an indigestible saccharide and the like to obtain the composition.

### The Invention Aspect 10

The subject of the present invention is to provide a whey protein food having stronger intestine-regulating effect.

The present inventors made a strenuous study and, as a result, found that whey protein, oligosaccharide and water-soluble dietary fiber are simultaneously ingested to give stronger intestine-regulating effect, thus achieving the present invention.

Therefore, the present invention is the following:
1) A whey protein food comprising whey protein, oligosaccharide and water-soluble dietary fiber.
2) The whey protein food according to 1) described above, having a granular form.

According to the invention described in claim 54, Whey protein itself has intestine-regulating effect, and can be simultaneously ingested with oligosaccharide and water-soluble dietary fiber to exhibit their synergistic effect, and to provide a food having stronger intestine-regulating effect. According to the invention described in claim 55, in addition, an excellently dispersible whey protein food can be provided to mix with the other foods to ingest.

Milk as a material for preparing whey protein for use in the present invention may be derived from any mammal such as cow, goat, sheep and horse. Further, the milk may either be collected from a vaccinated mammal or an unvaccinated mammal.

As the whey protein derived from these milk, commercially available usual ones derived from cow milk, i.e., those in the invention aspect 4 described above can be used.

Some whey protein contain remaining antibody derived from raw milk of material. Such antibody contained in whey protein also contributes to intestine-regulating effects of the whey protein food of the present invention. Therefore, whey protein for use in the present invention preferably comprises 0.5% by mass or more of the antibody.

The oligosaccharide for use in the present invention includes disaccharides to decasaccharides which are hardly digestible with digestive enzymes in human body, and any oligosaccharide can be used if it is usually used for food. As the examples, fructo-oligosaccharide, lactosucrose, lactulose, malt-oligosaccharide, isomalt-oligosaccharide, soybean-oligosaccharide, galacto-oligosaccharide, xylo-oligosaccahraide, raffinose, stachyose, palatinose-oligosaccharide, nigero-oligosaccharide, gentio-oligosaccharide, trehalose, the coupling sugar, pectin-oligosaccharide, alginic acid oligosaccharide, guar gum oligosaccharide, fucose oligosaccharide, cyclodextrin and the like can be mentioned.

In the present invention, dietary fiber means a non-digestible carbohydrate which can not be degraded with digestive enzymes in human body and in which saccharides with more than ten molecules are bound, and it is discriminated from indigestible glucide. The dietary fibers are broadly divided into water-soluble and water-insoluble dietary fibers, and in the present invention, water-soluble dietary fiber is used. As the water-soluble dietary fiber, pectin, guar gum, psyllium, galactomannan, xyloglucan, locust bean gum, Glucomannan, polydextrose, soluble hemicellulose, sodium alginate, chondroitin sulfuric acid, low molecular alginic acid, low molecular guar gum, fiberon and the like can be mentioned. Further, food materials comprising these water-soluble dietary fiber, for example, agar, bean-processed products, potato-processed products may be used.

The whey protein food of the present invention preferably comprises 5 to 1,000 parts by mass of oligosaccharide and 5 to 1,000 parts by mass of water-soluble dietary fiber based on 100 parts by mass of the whey protein. The contents of oligosaccharide and water-soluble dietary fiber of less than 5 parts by mass give insufficient intestine-regulating effect. Meanwhile, the contents exceeding 1,000 parts by mass lower so much the content of whey protein that the object of ingesting whey protein is difficult to achieve.

Further, the whey protein food of the present invention is granulated to improve so much the dispersibility of the whey protein food that the food can be easily mixed with the other food, in particular, a high water content food for ingestion. The whey protein is poor in water dispersibility, but in the present invention, is supplied with oligosaccharide and dietary fiber to improve the dispersibility to some extent compared with the whey protein alone. However, the whey protein is granulated and added in the other food such as cow milk and yogurt to improve further the dispersibility with no "floating mass" generated, and can provide a well-mixed food.

It is believed that the oligosaccharide described above serves as a binder in granulation, however, CMC (carboxymethyl cellulose), glucide and the like may further be added as a binder. Further, the granulation can be conducted by a common method such as spray granulation and extrusion granulation. There is no limit in the granule size, but the mean granule size is preferred to be from around 0.02 to 2 mm.

The present whey protein food can also comprise materials usually added in food such as sweetener, acidulant, flavor and polysaccharide thickening agent.

There is no limit in the form of the whey protein food of the present invention. For example, besides granules described above, it may be tablet, confetti, powder, capsule, and also past and liquid. The whey protein food can be any form of processed food unless the whey protein is added and then heated to inactivate the antibody during production of the food.

### The Invention Aspect 14

The present inventors have screened various food materials to solve the problem described above and, as a result, found that whey protein is supplied with a powder selected from green tea, aloe, turmeric, pumpkin, red grape juice and tomato powders to reduce milk smell and puckery and astringent tastes possessed by whey protein, and to improve so much the water absorption that the whey protein is mixed with a liquid food such as cow milk or a pasty food such as yogurt to generate a "floating mass" which can be disentangled in a short time, and that the whey protein is colored with a pigment derived from a natural material to provide a tasty and a pleasant feeling, thus achieving the present invention.

Therefore, the present invention is the following:
1) A whey protein food comprising whey protein and 1 to 900 parts by mass of a powder selected from green tea, aloe, turmeric, pumpkin, red grape juice and tomato powders based on 100 parts by mass of the whey protein.
2) The whey protein food according to claim 56, having a granular form.

According to the present invention, the whey protein food is supplied with a defined amount of powder selected from green tea, aloe, turmeric, pumpkin, red grape juice and tomato powders to reduce milk smell and puckery and astringent tastes possessed by whey protein decrease, and to improve so much flavor and the water absorption that the food is mixed with a liquid or pasty food to generate a floating mass which can be easily disentangled. Further, green tea powder, green color of aloe powder, turmeric powder, yellow color of pumpkin powder, red grape juice powder, red color of tomato powder visually attracts an ingesting person, induces appetite, and offers fun and a change. The whey protein food is granulated to improve the water absorption greatly, allowing an easier mixing with the other liquid or pasty food and an easier tasting.

In the present invention, for whey protein, commercially available usual products and those in invention aspect 4 described above can be used.

Milk as a material for preparing whey protein for use in the present invention may be derived from any mammal such as cow, goat, sheep and horse. Further, the milk may either be collected from a vaccinated mammal or an unvaccinated mammal.

In the present invention, as green tea powder, a commercially available usual product can be used. Whey protein is supplied with green tea powder, allowing the bitterness of green tea powder to erase milk smell and puckery and astringent tastes possessed by whey protein, and enabling the flavor and green color of green tea powder to provide the whey protein food with tastier feeling. Further, the whey protein is supplied with green tea powder to improve its water absorption, providing easy mixing with a liquid or pasty food. In particular, the flavor and color of green tea powder attracts the elderly for their palatability, to give a preferable whey protein food.

In the present invention, as aloe powder, a commercially available usual product can be used. The whey protein food is supplied with aloe powder, allowing the bitterness of aloe powder to erase milk smell and puckery and astringent tastes possessed by whey protein, and enabling the flavor and green color of aloe powder to provide the whey protein food with tastier feeling. The whey protein is supplied with aloe powder to improve its water absorption, providing an easy mixing with a liquid or pasty food.

In the present invention, as turmeric powder, a commercially available usual product can be used. The whey protein food is supplied with turmeric powder, allowing the bitterness of turmeric powder to erase milk smell and puckery and astringent tastes possessed by whey protein, and enabling the flavor and yellow color of turmeric powder to provide the whey protein food with tastier feeling. Further, its improved water absorption allows an easy mixing with a liquid or pasty food.

In the present invention, as pumpkin powder, a commercially available usual product can be used. The whey protein food is supplied with pumpkin powder, allowing the faint sweetness of pumpkin powder to erase milk smell and puckery and astringent tastes possessed by whey protein, and enabling the flavor and yellow color of pumpkin powder to provide the whey protein food with tastier feeling. Further, its improved water absorption allows an easy mixing with a liquid or pasty food.

A red grape juice powder for use in the present invention can be produced from a commercially available usual grape juice. There is no limit in red grape for the material. Whey protein is supplied with red grape juice powder, allowing the tannin component of red grape juice powder to erase milk smell and puckery and astringent tastes possessed by whey protein, and enabling the flavor and red color of red grape juice powder to provide the whey protein food tastier feeling. Further, its improved water absorption allows easy mixing with a liquid or pasty food.

Further, as tomato powder for use in the present invention, a commercially available usual product can be used. Whey protein is supplied with tomato powder, allowing the unique taste of tomato powder to erase milk smell and puckery and astringent tastes possessed by whey protein, and enabling the flavor and red color of tomato powder to provide the whey protein food with tastier feeling. Further, the whey protein is supplied with tomato powder to improve its water absorption, allowing an easy mixing with a liquid or pasty food.

The whey protein food of the present invention comprises 1 to 900 parts by mass of powder selected from green tea, aloe, turmeric, pumpkin, red grape juice and tomato powders based on 100 parts by mass of the whey protein. The content of powder described of less than 1 parts by mass gives its insufficient effects for reducing milk smell and puckery and astringent tastes possessed by whey protein and for improving the water absorption, and provides insufficient coloring. Meanwhile, the content exceeding 900 parts by mass lowered so much the content of the whey protein that the object of ingesting whey protein is difficult to achieve.

Further, the whey protein food of the present invention is preferably granulated as in the invention aspect 4 described above, allowing easy mixing with a liquid or pasty food.

It is believed that the powders described above contain glucide, starch and fiber derived from their respective plants, so these components serves as a binder in granulation, however CMC (carboxymethyl cellulose), glucide and the like may be added as a binder. Further, the granulation can be conducted by a common method such as spray granulation and extrusion granulation.

The whey protein food of the present invention can also comprise materials usually added in food such as sweeteners such as sucrose, erythritol and Stevia extract, acidulants such as citric acid and malic acid, flavor and polysaccharide thickening agent.

The whey protein food of the present invention has advantages in the same manner as described above.

### The Invention Aspect 5

The subject of the present invention is to provide a method for producing whey protein, wherein only current production equipment is used to protect an antibody from heat inactivation during a production process with a high percentage of remaining antibody allowed to attain.

The present inventors made s strenuous study and, as a result, found that a glucide can be added during a process for producing whey protein to suppress the antibody from being inactivated by heat, and that only current production equipment can be used to increase the percentage of remaining antibody, thus achieving the present invention.

Therefore, the present invention is the following:
1) A method for producing whey protein, wherein a glucide is added at least at any time point during a process for producing the whey protein to increase the percentage of remaining antibody.
2) The method for producing whey protein according to 1) described above, wherein the glucide is added before raw milk is heated to sterilize.
3) The method for producing whey protein according to 1) described above, wherein the glucide is added before the whey is heated.
4) The method for producing whey protein according to claim 59 or 3) described above, wherein the glucide is lactose.
5) The method for producing whey protein according to 1) described above, wherein the glucide is added before the whey protein is dried.
6) The method for producing whey protein according to 5) described above, wherein the glucide is an indigestible saccharide.
7) The method for producing whey protein according to 1) to 6) described above, wherein the percentage of remaining antibody is 70% or more of an antibody contained in material milk.

The present invention provides a method for producing whey protein, wherein only current production equipment is used, and a glucide can be added during a process for producing the whey protein to increase the heat stability of an antibody and to suppress the antibody from heat-inactivation, resulting in a high percentage of remaining antibody which has been contained in raw milk. This effect can be attained by adding lactose as the glucide before raw milk is heated to sterilize or before the whey is heated, and the lactose thus added can be collected in a step for separating the lactose. Further, an indigestible saccharide can be added as the glucide before the whey protein is dried, to increase the stability of the antibody and to produce a whey protein which is expected to have a synergistic effect between immunostimulatory function possessed by the antibody and physiological function possessed by the indigestible saccharide. Further, because the whey protein thus obtained holds as much as 70% or more of the antibody which has been contained in raw milk used as a material, the whey protein may be ingested by the least amount necessary to take a specific amount of the antibody. The whey protein allows easy ingestion of the antibody.

Whey protein is a protein contained in whey obtained as a byproduct in producing cheese and casein from animal milk or defatted milk, and usually produced through various steps as shown in Fig. 2. Fig. 2 shows a typical step. The production process of whey protein of the present invention is not limited to the steps shown in Fig- 2. The process includes, for example, a step wherein sterilized milk is inoculated with lactobacilli to ferment, and supplied with Rennet to separate a solidified curd (cheese) from cheese whey. Whey is referred as milk serum, and whey is described in Fig. 2. The current production equipment for whey protein described above includes equipment which is usually used for the production of WPC or WPI as shown in Fig. 2, and does not include special equipment such as a chromatography apparatus which is used to enhance the antibody concentration.

In the present invention, the raw milk as a material for preparing whey protein may be milk of any mammal such as cow, goat, sheep, horse and buffalo, and further, the raw milk may either be collected from a vaccinated mammal or an unvaccinated mammal. Preferably, 70% or more of an antibody contained in these raw milk keep its activity to remain in whey protein.

The present invention is characterized in that a glucide is added at least at any time point in the production process of whey protein to increase the percentage of remaining antibody. The other step than a step for adding a glucide follows a conventional method for producing whey protein as shown in Fig. 2. In the present invention, as glucide to add, if it is useful for food, any glucide such as lactose, indigestible saccharide, glucose, fructose, sucrose, isomerized sugar and dextrin may be used. There is no limit in a method for adding these glucide. The glucide is added to protect an antibody from heat-inactivation with an increased amount of remaining antibody contained in the whey protein.

The amount of the glucide to add is preferably set to have a glucide content of 6.5% by mass or more including an originally contained glucide, and more preferably, 6.5 to 12.5% by mass. For example, because a glucide is originally contained in raw milk at 4.5%, the amount of the glucide to add in the raw milk is most desirably 2 to 8% by mass. The glucide content of less than 6.5% by mass gives an insufficient effect for reducing the loss of antibody. Meanwhile, the content exceeding 12.5% by mass can precipitate a glucide depending on the kind of the glucide.

Fig. 3 shows the percentage of remaining antibody when 0 to 8% by mass of lactose were added in raw milk and heated for 30 minutes at 65°C. In Ministerial ordinances covering milk (ordinances relating to the component and standard of milk and dairy product), the regulation for the thermal sterilization of raw milk defines that raw milk should be heated to sterilize at 63°C for 30 minutes, or by a way for attaining the equivalent or higher sterilization effects. 4.5% by mass of lactose is contained in raw milk, so the horizontal axis shows a lactose content of this amount. No lactose is added to give a percentage of remaining antibody of less than 80%, while lactose is added to improve the heat stability of antibody, allowing inhibition of the antibody from loss in a sterilization step with an increased percentage of remaining antibody contained in the whey protein thus obtained.

In the present invention, the glucide may be added in any step during the production process of whey protein as described above, and is preferably added prior to a step involving heating. Specifically, the glucide is preferably added at any time point shown by 1 to 9 in Fig. 2 (shown in circled number in Fig. 2., same hereinafter). The timing of adding glucide and the kind of preferable glucide will be explained below to follow the steps.

Firstly, glucide can be added before raw milk is heated to sterilize, as shown at 1 in Fig. 2. In this case, the glucide is added after raw milk is washed to adjust components and before the raw milk is heated to sterilize by a method such as HTST sterilization (high-temperature short-time sterilization, at 72 to 75°C for 15 sec.), or LTLT sterilization (low-temperature long-time sterilization, at 65°C for 30 min.) .

Sterilized milk can be used to produce cheese, casein curd or Rennet casein and the like by a usual method. Whey such as cheese whey, acid whey or Rennet casein whey is separated from them to obtain.

Mainly in the case wherein no glucide is added at the timing of 1 described above, the glucide may be added at the next timing before the separated whey (whey in Fig. 2) is heated if needed (2, 3 and 4 in Fig. 2).

Lactose is used as the glucide added (at any timing shown by 1 and 2 to 4 in Fig. 2) as described above, allowing inhibition of an antibody from heat-inactivation as well as collection of the added lactose together with lactose contained in raw milk in a succeeding step. This is preferable because of its good cost performance.

Then, whey protein solution is separated from whey (solution) which has been heated if needed, and there is no limit in the separation or fractionating method. For example, ultrafiltration, ion-exchange, microfiltration and the like may be mentioned. Further, if needed, it can be concentrated.

Further, there is no limit in the method for drying the whey protein solution. For example, spray-drying, freeze-drying and the like can be mentioned. Glucide may be added before this drying (timing shown at 5, 6, 7, 8 and 9 in Fig. 2). The glucide is added, allowing inhibition of an antibody from the loss during drying.

An indigestible saccharide is preferably used as the glucide added before a whey protein solution is dried. The indigestible saccharide becomes a growth factor for intestinal bifidobacteria, and those bifidobacteria affect immune system in gastrointestinal tract, thereby activating active immunity. Meanwhile, an antibody contained in whey protein activates passive immunity. Therefore, both an indigestible saccharide and whey protein are ingested to activate both active immunity and passive immunity, thus strong immunostimulatory effects can be expected to obtain. Further, some indigestible saccharides have such functions as intestine-regulating effect, mineral absorption facilitating effect, anti-caries effect, effect for inhibiting the increase of blood cholesterol, and effect for improving liver function, and are expected to exhibit them together with the immunostimulatory effect. Therefore, the whey protein with an increased percentage of remaining antibody and the indigestible glucide can be ingested to give an expected synergistic effect between their respective effects described above.

For the indigestible saccharide, those of the invention aspect 4 described above may be used.

### Examples

The present invention will further be explained in detail below with respect to Examples, but the present invention will not be limited to them.
The numbers of Examples correspond to the numbers of the aspects of the invention described above.

### [Examples 1-1 to 1-5 and Comparative Example 1-1]

5 kinds of foods comprising whey protein and cocoa and a food comprising whey protein but cocoa powder for comparison (Comparative Example 1-1) were prepared according to the compositions shown in Table 1-1 described below (numerical values represent mass ratio). In Table 1, Proliant® 8000 is a whey protein (from WPC, James Farrell & Co.) .

**[Table 1-1]**

| | Comparative Example 1 | Example 1 | Example 2 | Example 3 | Example 4 | Example 5 |
|---|---|---|---|---|---|---|
| Proliant 8000 | 100 | 100 | 90 | 50 | 10 | 1 |
| Cocoa powder | - | 1 | 10 | 50 | 90 | 100 |

| | | | | | | |
|---|---|---|---|---|---|---|
| (Evaluating improvement of flavor) | | | | | | |

20 g of each prepared whey protein/cocoa food and 6 g of sucrose were added in 100 ml of a commercially available ultra high temperature sterilized cow milk (hereinafter, abbreviated as "cow milk") heated to 65°C and homogeneously mixed to get a sample, for which sensory evaluation was performed by 10 expert panelists according to 5-point scale. Further, 20 g of each whey protein/cocoa food and 6 g of sucrose were added in 100 ml of cold water and homogeneously mixed to get a sample, for which evaluation was also performed. Their average scores are shown in Table 1-2. A higher score means better flavor. Every Example showed high scores compared with Comparative Examples, thus all 5 kinds of foods comprising whey protein and cocoa were improved in flavor of whey protein.

**[Table 1-2]**

| | Comparative Example 1 | Example 1 | Example 2 | Example 3 | Example 4 | Example 5 |
|---|---|---|---|---|---|---|
| Evaluation in cow milk | 1.6 | 3.9 | 4.0 | 4.2 | 4.6 | 4.4 |
| Evaluation in cold water | 1.4 | 3.8 | 3.9 | 4.1 | 4.3 | 4.2 |

Meanwhile, in Examples 1-1 to 1-5, whey protein / cocoa foods supplied with cocoa powder was added together with sucrose in 65°C cow milk and cold water. They all hardly formed "floating mass", compared with a powder comprising whey protein alone, indicating an improved water absorption.

### [Example 1-6]

40 g of whey protein/cocoa food having a composition shown in Examples 1-5 and 12 g of sucrose were added in 200 ml of cow milk at 65°C, to form little "floating mass", which was easily dispersed and mixed. In addition, a cup of the product was ingested tastefully.

### [Example 1-7]

A whey protein/cocoa food having a composition shown in Example 1-1 was granulated by spray granulation. 40 g of this granular whey protein/cocoa food was added in 200 ml of cold cow milk. The product quickly absorbed water, and was dispersed and mixed.

### [Examples 2-1 to 2-4, Comparative Example 2-1]

5 kinds of whey protein foods were prepared in their respective compositions shown in Table 2-1 described below (numerical values represent mass ratio). In Table 2-1, Proliant® 8000 is a whey protein (from WPC, James Farrell & Co.). Further, cranberry, raspberry, blueberry and strawberry powders are all from Tokiwa Phytochemical Co., Ltd.

**[Table 2-1]**

| | Comparative Example 2-1 | Example 2-1 | Example 2-2 | Example 2-3 | Example 2-4 |
|---|---|---|---|---|---|
| Proliant8000 | 100 | 90 | 50 | 99 | 10 |
| Cranberry powder | - | 10 | - | - | - |
| Raspberry powder | - | - | 50 | - | 45 |
| Blueberry powder | - | - | - | 1 | - |
| Strawberry powder | - | - | - | - | 45 |

| | | | | | |
|---|---|---|---|---|---|
| (Evaluating improvement of flavor) | | | | | |

5 parts by mass of whey protein food prepared was added in 95 parts by mass of a commercially available plain yogurt and homogenized to get one sample, and similarly added in 95 parts by mass of a commercially available ultra high temperature sterilized cow milk (hereinafter, abbreviated as "cow milk") and homogenized to get another sample. For these samples, sensory evaluation was performed by 10 expert panelists according to 5-point scale. Their average scores are shown in Table 2-2. A higher score means better flavor. The Examples all receive high scores, thus 4 kinds of whey protein foods all were improved in flavor of whey protein.

**[Table 2-2]**

| | Comparative Example 2-1 | Example 2-1 | Example 2-2 | Example 2-3 | Example 2-4 |
|---|---|---|---|---|---|
| Flavor of yogurt | 1.6 | 4.6 | 4.4 | 4.3 | 4.5 |
| Flavor of cow milk | 1.4 | 4.3 | 4.1 | 4.0 | 4.3 |

Meanwhile, in these Examples, when whey protein foods, which had been supplied with cranberry, raspberry, blueberry and strawberry powders, were added to yogurt to drink, they all hardly formed a "floating mass" compared with a powder comprising whey protein alone, indicating that they were improved in water absorption.

### [Examples 2-5 to 2-8]

Whey protein foods having compositions shown in Examples 2-1 to 2-4 were formed into a granular whey protein food by spray granulation. When 5 parts by mass of these granular whey protein foods were added in 95 parts by mass of cow milk respectively, they all quickly absorbed water, precipitated in the cow milk, and could be dispersed to mix.

### [Example 2-9]

When 4 g of whey protein food having compositions shown in Example 2-4 was added in 100 g of yogurt, "floating masses" were hardly formed, and easily dispersed and mixed. The whole cup could be ingested tastefully.

### [Example 3-1]

Milactele 80 (From Morinaga Milk Industry Co., Ltd.), which is a whey protein, was diluted 10 times with an equal-amount mixture of sucrose and CMC (carboxymethyl cellulose), granulated using a fluid bed granulator with the product temperature adjusted to 65°C, and used to test the following effects as a fecal odor-reducing agent. The antibody content of this granule was 0.5% by mass.

### (Effect testing)

As subjects, twenty men and women (12 in 21-30 years old, 4 in 31-40 years old and 4 in 41-60 years old) were divided into four groups (5 in each group). Test group 1 was served with 5 g (antibody of 25 mg) of the granule obtained in Example 1 (fecal odor-reducing agent), test group 2 was served with 10 g (antibody of 50 mg) of the same granule, and test group 3 was served with 20 g (antibody of 100 mg) of the same granule, which were suspended in 100 ml of water to drink after every breakfast respectively. Further, a control group was served with 10 g of an equal-amount (mass) powder of sucrose and CMC which was suspended in 100 ml of water to drink after every breakfast.

Then, they performed 5-grade sensory evaluation (0: almost no smell, 1: slight smell, 2: usual smell, 3: strong smell, 4: extremely strong smell) on their fecal odor before and the 3rd and 7th days after the initiation of drinking, and their mean values were calculated. The results were shown in Table 1.

**[Table 3-1]**

| | Before ingestion | 3rd day after | 7th day after |
|---|---|---|---|
| Test Group 1 (25 mg of antibody) | 2.8 | 2.2 | 1.8 |
| Test Group 2 (50 mg of antibody) | 2.6 | 1.4 | 0.6 |
| Test Group 3 (100 mg of antibody) | 2.8 | 1.0 | 0.4 |
| Control Group | 2.6 | 2.8 | 2.6 |

From Table 3-1, it can be known that fecal odor was improved in each test group. In particular, it is shown that effect of improving fecal odor is evidently high in test groups 2 and 3. Meanwhile, during the testing period, smell of gas was also improved in many of subjects in test groups, and so was bowel movement.

### [Example 3-2]

8 parts by mass of Milactele 80 (From Morinaga Milk Industry Co., Ltd.), which is a whey protein, and 2 parts by mass of Meioligo CR (from Meiji Seika Kaisha, Ltd.), which is fructo-oligosaccharide, were mixed, granulated using a fluid bed granulator with the product temperature adjusted to 65°C, and used to test the following effects as a fecal odor-reducing agent. The antibody content of this granule was 4.0%.

### (Effect testing)

As subjects, four men who were particularly concerned about their strong fecal odor(from 61 to 67-year old) were selected and allocated to test group 4, and then served with 5 g (antibody of 200 mg) of the obtained granule from Example 3-2 (fecal odor-reducing agent) which was suspended in 100 ml of water to drink after every breakfast. Then, 5-grade sensory evaluation was performed on fecal odor before and the 3rd and 7th days after the initiation of drinking in the same manner as Effect testing in Example 3-1. Those mean values are shown in Table 3-2, and the individual evaluation points are put into a graph as shown in Fig. 1.

**[Table 3-2]**

| | Before ingestion | 3rd day after | 7th day after |
|---|---|---|---|
| Test Group 4 (200mg of antibody) | 4.0 | 2.25 | 1.25 |

Higher effects of improving fecal odor are obtained in persons who were more concerned about their strong fecal odor, as can be seen from comparing Table 3-2 and Fig. 1 with Table 3-1. In particular, for one of them, by taking 200 mg of antibody for seven days, fecal odor was improved from level (4) of extremely strong smell to level (0) of almost no smell.

The present invention will be explained in more detail below with respect to Examples, but the present invention will not be limited to them. In the Examples, the amount of antibody was measured by the following method.

### (Method for Determining Amount of Antibody)

100 mg of granulated whey protein food comprising whey protein or antibody was dissolved in 50 ml of 50 mM phosphate buffer (pH 6.8), and stirred for more than 2 hours to get a solution, 10 ml or more of which was then filtered through a 0.45 µ-membrane filter.

Meanwhile, protein G column (HiTrap Protein G HP 1 ml, Amersham) was equilibrated with 50 mM phosphate buffer (pH 6.8) , and 10 ml of the sample, which had been filtered through the membrane filter, was applied to the column and washed with the same buffer (pH 6.8), and then the antibody was eluted with 100 mM glycine-hydrochloride buffer (pH 2.7) .

The absorbance of the eluate was measured at 280 nm, then the antibody concentration of the sample was determined on the assumption that the absorbance of antibody having a concentration of 1% is 14, and then the amount of antibody in the granulated whey protein food comprising whey protein or antibody was calculated.

### [Reference Example 4-1]

### (Determination of Antibody Content of Whey Protein)

According to the determination method described above, antibody contents of commercially available whey proteins were measured. Those of Milactele 80 (Morinaga Milk Industry Co., Ltd.), TATUA 902 (Tatua Japan K. K.), and Proliant 8000 (James Farrell & Co.) were 50 mg/g, 42 mg/g, and 55 mg/g respectively.

### [Examples 4-1 to 4-5]

5 kinds of whey protein foods with compositions shown in Table 1 described below (numbers represent mass ratio) were prepared, and their antibody contents (mg) in per gram were measured. In Table 4-1, Proliant^{®} 8000 is a whey protein (WPC, from James Farrell & Co.), Meioligo^{®} P is fructo-oligosaccharide (from Meiji Seika Kaisha, Ltd.), Oligomate^{®} 55P is galacto-oligosaccharide not having α-galactosyl binding (from Yakult Pharmaceutical Ind. Co., Ltd.), Nyukaoligo^{®} LS-55P is lactosucrose (from ENSUIKO Sugar Refining Co., Ltd.), milk oligo is lactulose (From Morinaga Milk Industry Co., Ltd.) and isomalt^{®} 900P is isomalt-oligosaccharide (from Nikken Fine Chemicals Co., Ltd.).

**[Table 4-1]**

| | Comparative Example | Example 4-1 | Example 4-2 | Example 4-3 | Example 4-4 | Example 4-5 |
|---|---|---|---|---|---|---|
| Proliant8000 | 100 | 95 | 90 | 80 | 50 | 10 |
| Meioligo P | - | 5 | - | - | - | - |
| Oligomate 55P | - | - | 10 | - | - | - |
| Nuykaoligo LS-55P | - | - | - | 20 | - | - |
| Milk oligo | - | - | - | - | 50 | - |
| Isomalt 900P | - | - | - | - | - | 90 |

| | | | | | | |
|---|---|---|---|---|---|---|
| (Evaluating improvement of flavor) | | | | | | |

10 parts by mass of each whey protein food prepared was added in 90 parts by mass of commercially available plain yogurt and homogenized to get one sample, and similarly added in 90 parts by mass of a commercially available ultra high temperature sterilized cow milk (hereinafter, abbreviated as "cow milk") and momogenized to get another sample. For these samples, sensory evaluation was performed according to 5-grade (1: bad, 2: rather bad, 3: usual, 4: rather good, 5: good) by 10 expert panelists, and their mean values were calculated. Their average scores are shown in Table 2. The Examples all receive high scores, thus 5 kinds of whey protein foods all were improved in the flavor of whey protein.

**[Table 4-2]**

| | Comparative Example | Example 4-1 | Example 4-2 | Example 4-3 | Example 4-4 | Example 4-5 |
|---|---|---|---|---|---|---|
| Flavor of yogurt | 1.2 | 4.6 | 4.5 | 4.5 | 4.6 | 4.6 |
| Flavor of cow milk | 1.1 | 4.5 | 4.2 | 4.3 | 4.3 | 4.4 |

Meanwhile, in these Examples, when whey protein foods supplied with fructo-oligosaccharide, lactosucrose, lactulose, isomalt-oligosaccharide and galacto-oligosaccharide with no α-galactosyl bond contained were added in yogurt to drink, they all hardly formed a "floating mass", compared with a powder comprising whey protein alone, indicating that they were improved in water absorption and had better dispersibility.

### [Examples 4-6 to 4-8]

Whey protein foods having compositions shown in Examples 4-1, 4-2 and 4-3 were granulated into granular whey protein foods using a fluid bed granulator with the product temperature adjusted to 65°C. When 10 parts by mass of these granular whey protein foods were added in 90 parts by mass of cow milk, they extremely quickly absorbed water, precipitated in the cow milk and could be dispersed.

### [Example 4-9]

A whey protein solution was obtained by concentrating a cheese whey (protein content, 0.6%) and separating crystallized lactose. 10 parts by mass of Toreha^{®} (trehalose from Hayashibara Shoji, Inc.) was added thereto based on 100 parts by mass of whey protein as solid content, and spray-dried to obtain a powder of whey protein food having an antibody content of 55 mg/g. When 5 g of this whey protein food was added in 100 g of tomato juice, it was quickly dissolved therein to produce a tomato juice comprising 275 mg of antibody. Any milk smell, acrid taste and the like were not felt in the flavor of this tomato juice.

### [Example 4-10]

100 parts by mass of Milactele 80, 20 parts by mass of inulin of Fuji FF (from Fuji Nihon Seito Corporation), 2 parts by mass of polyglutamic acid (from Meiji Seika Kaisha, Ltd.), and 1 parts by mass of succinic acid monoglyceride were mixed and granulated using a fluid bed granulator with the product temperature adjusted to 65°C to obtain a granular whey protein food having an antibody content of 40 mg/g. When 5 g of this granular whey protein food was dispersed in 100 g of yogurt, it was quickly and uniformly mixed, providing a yogurt comprising 200 mg of antibody. Any acrid taste and the like were not felt in the flavor of this yogurt.

### [Example 4-11]

100 parts by mass of TATUA 902 and 5 parts by mass of erythritol were mixed and granulated using a fluid bed granulator with the product temperature adjusted to 70°C, providing a granular whey protein food comprising an antibody content of 38 mg/g. When 5 g of this granular whey protein food was added in 100 g of iced coffee, it was quickly dissolved therein to produce a coffee beverage comprising 190 mg of antibody. Any acrid taste and the like were not felt in the flavor of this coffee beverage.

### [Example 4-12]

100 parts by mass of Proliant 8000 and 1000 parts by mass of Meioligo^{®} CR (from Meiji Seika Kaisha, Ltd. fructo-oligosaccharide) were mixed to obtain a whey protein food having an antibody content of 5 mg/g. When 5 g of this whey protein food was dispersed in 100 g of soy milk, it was quickly and uniformly mixed, providing a soy beverage comprising 25 mg of antibody. Any milk smell, acrid taste and the like were not felt in the flavor of this soy beverage.

### (Method for Determining Amount of Antibody)

An adsorbed fraction which had been obtained from an anti-bovine IgG rabbit immune serum (from YAGAI Corporation) with protein G column, was used as primary antibody, and HRP labeled anti-bovine IgG antibody (from Cosmo Bio Co. , Ltd.) was as secondary antibody, to conduct determination by ELISA according to a common method. As an antibody preparation, a protein G-adsorbed fraction was obtained by conducting thrice-repeated adsorption/desorption of whey powder obtained from cow's colostrum using protein G column according to a conventional method, and then a low molecular fraction was removed by dialysis to get protein G-adsorbed fraction, which was then used to prepare a standard curve for determining the antibody concentration of the sample.

### [Example 5-1]

100 L of raw milk (milk fat content 3.4%, antibody content 0.21 mg/ml) was supplied with 2.0 kg of lactose, and heated at 72°C for 15 seconds to sterilize. Then, according to a common method, it was centrifuged to obtain a nonfat milk, which was supplied with lactic acid to acidify, squeezed to separate casein curd. Acid whey thus obtained was ultrafitrated to separate lactose and spray-dried to obtain whey protein (WPC). The antibody content of this whey protein was 57 mg/g, indicating that 70% of antibody remained in terms of the amount of antibody contained in the material milk.

### [Comparative Example 5-1]

The raw milk used in Example 5-1 was processed to obtain whey protein (WPC) in the same way as in Example 5-1 without lactose added. The antibody content of this whey protein was 15 mg/g, indicating that only 19% of antibody remained in terms of the amount of antibody contained in the material milk.

### [Example 5-2]

Raw milk was heated at 72°C for 15 seconds to sterilize. The sterilized milk thus obtained lost already a part of antibody, from which cheese was prepared and cheese whey (protein content, 0.8%) was obtained as a byproduct. 100 L of this cheese whey was supplied with 5.0 kg of lactose, washed at 65°C and ultrafiltrated at 53°C to separate lactose. The product was spray-dried to obtain whey protein (WPC). The antibody content of this whey protein was 70 mg/g.

### [Comparative Example 5-2]

The cheese whey used in Example 5-2 was processed in the same way as in Example 5-2 without lactose supplied to obtain whey protein (WPC). The antibody content of this whey protein was 38 mg/g.

### [Example 5-3]

A whey protein solution was obtained by ion-exchange from the cheese whey used in Example 5-2. 100 L of this whey protein solution was supplied with 8.0 kg of fructo-oligosaccharide and spray-dried to obtain whey protein (WPI) . The antibody content of this whey protein was 62 mg/g.

### [Comparative Example 5-3]

The cheese whey used in Example 5-2 was processed in the same way as in Example 3 without fructo-oligosaccharide supplied to obtain whey protein (WPI). The antibody content of this whey protein was 9 mg/g.

### [Example 5-4]

Raw milk was heated at 65°C for 30 minutes to sterilize. The obtained sterilized milk already lost a part of antibody, from which casein curd was prepared according to a conventional method and acid whey (protein content, 0.9%) was obtained as a byproduct. A whey protein solution was obtained from this acid whey by ultrafiltration (this whey protein solution contained lactose at 3.5 % by mass). 100 L of this whey protein solution was supplied with 3.0 kg of trehalose, and spray-dried to obtain whey protein (WPC) . The antibody content of this whey protein was 60 mg/g.

### [Comparative Example 5-4]

The acid whey used in Example 5-4 was processed in the same way as in Example 5-4 without trehalose supplied to obtain whey protein (WPC) . The antibody content of this whey protein was 42 mg/g.

### [Example 6-1]

a) Immunization of cow with collagen
   2 mg of type II chicken collagen (Chondrex. Inc) was dissolved in 1 ml of 0.05 M acetic acid to prepare 2 mg/ml acetic acid solution of collagen. The same volume of Phosphate Buffer Saline (PBS) having twice the concentration of that was added to make up to 2 ml, 1 ml of which was supplied with 1 ml of TiterMax gold and sufficiently stirred to prepare an emulsion. 4 pregnant cows were immunized with 2 ml of the emulsion, each 1 ml of which was subcutaneously injected at two different places in the cervical area, 4 times on 60 and 30 days before and 30 and 60 days after their respective expected delivery dates.
b) Immunization of cow with E. coli 0-111: B4
   E. coli O-111 B4 was cultured and proliferated on a nutrient agar plate medium and scraped after the addition of 1% formalin-saline, and then heated to sterilize at 80°C for an hour. After washing the heated bacterial culture, turbidity was adjusted to an absorbance of 1.0 0 at a wavelength of 660 nm. 1 ml of the bacterial culture was added to 1 ml of TiterMax gold and efficiently stirred to prepare an emulsion. 3 pregnant cows were subcutaneously injected with each 1 ml of the emulsion in the cervical area two times on 60 and 30 days before the expected delivery date.
c) Preparation of Whey from Colostrum of Cow Immunized with Chicken Type II collagen and from those with E. coli O-111 B4

Raw milk was collected to pool from each cow immunized in a) and b) described above for three days after delivery. 8 liters of the raw milk was centrifuged at 20000 g for 30 minute to remove fat layer, thereby to get nonfat milk. The nonfat milk was heated to sterilize at 62.5°C for 30 minutes and supplied with hydrochloric acid to adjust pH to 4.6, centrifugated at 20000 g for 30 minute to remove casein, supplied with sodium hydroxide solution to adjust pH to 7.0 and freeze-dried to give dry products of 707 g and 780 g respectively.

### [Example 6-2]

### Specific Purification of Anti-LPS Bovine Antibody

Whey from E. coli immunized cows prepared in Example 1 c) contained anti-E. coli endotoxin O-111 antibody at 0.25%. This whey was added in phosphate-buffered saline 501 to prepare a 1% solution, which was applied on 500 ml O-111 immobilized affinity column, washed, elutedatpH2-8, neutralized, then further concentrated and purified using protein G column to obtain 580 mg of the antigen specific antibody.

### [Example 6-3]

### Specific Purification of Anti-chicken Type II Collagen Bovine Antibody

Whey prepared from colostrum of cows immunized with chicken Type II collagen prepared in Example 1 c) contained anti-chicken Type II collagen antibody at 0.6%. This whey was added in phosphate-buffered saline 301 to prepare a 1% solution, which was applied on 500 ml 0-111 immobilized affinity column, washed successively, eluted at pH 2.8, neutralized, further concentrated and purified using protein G column to obtain 930 mg of the antigen specific antibody.

### [Example 6-4]

Effects of anti-LPS bovine antibody for preventing the induction of arthritis on an arthritis model induced by the mixture of mouse anti-collagen and monoclonal antibody

Mouse is injected with 4 mg of monoclonal antibody to mouse articular cartilage collagen (arthritis cocktail, Chondrex. Inc) to induce arthritis, which led to maximum swelling in joint on day 2. While, when the amount of arthritis cocktail was reduced to 2 mg, no arthritis was induced. However, oral administration of E. coli LPS (E. coli O-111: B4 Sigma) to these mice three times at 3 mg/mouse/day (on day 0, 1 and 2 with the administration day of arthritis cocktail referred to as day 0) induced arthritis, which led to maximum swelling in day 6. Three groups, each of which was composed of three 6-week old BALB/c male mice, were set for: arthritis cocktail group, combination group of arthritis cocktail and orally-administrating LPS, administration group of arthritis cocktail, orally-administrating LPS and further anti-LPS antibody, and swelling of arthritis was evaluated by measuring both leg volume. Referring to anti-LPS bovine antibody, the purified specific antibody shown in Example 2 was ingested at 3 mg/mouse/day on LPS administration day. The results were shown in Table 6-1.

**[Table 6-1]**

| | Leg volume (ml ± SE) | | Significant difference to LPS administration group 2. (p) |
|---|---|---|---|
| Treatment group | Antibody administration day (day 0) | Day 6 | |
| Arthritis cocktail | 0.207 ± 0.004 | 0.206 ± 0.004 | |
| Arthritis cocktail + LPS ingested | 0.189 ± 0.005 | 0.257 ± 0.001 | |
| Arthritis cocktail + LPS ingested + anti-LPS antibody ingested | 0.195 ± 0.009 | 0.188 ± 0.005 | P<0.001 |

As shown in Table 6-1, arthritis inducing effects of orally-administrating LPS was prevented by oral administration of specifically purified anti-LPS antibody.

### [Example 6-5]

Prevention effects of anti-collagen antibody and anti-LPS antibody on induction of mouse arthritis by orally and long-term administration of chicken Type II collagen and LPS

Oral administration of chicken type II collagen mixed with LPS to mice enhances the antibody production and induction of arthritis compared with the administration group of type II collagen alone. (K. Terato et al., Br. J. Rheum. 35, 828-838, 1996). Therefore, antibodies were examined for respective inhibitory effects of on induction of arthritis using DBA/1 mice. Six arthritis-prone 6-week old DBA/1 male mice composed 1 group, and the following administration groups were set: collagen (chicken Type II collagen Chondrex. Inc) alone, collagen plus LPS (E. coli O-111: B4, Sigma), and anti-collagen antibody (Example 6-3) and anti-LPS antibody (Example 6-2) further added thereto. Oral immunity was conducted over 14 weeks through 4 courses, wherein two weeks made up 1 course. In oral administration for oral immunity, to facilitate the oral absorption of collagen and LPS, 40 µg of indomethacin was added to make a total volume of 0.4 ml in all groups including control group 1. Blood was collected from the ocular fundus to obtain serum on the 1st date of 16th week, LPS was then orally administer to induce arthritis for a week. On the 1st day of each immunization course and on the 1st day of 17th week, swelling in joint was scored (see literatures described in Table 6-2) and rated. The constituents of animal groups, treatment conditions, and volumes of footpads were shown in Table 6-2. Administration amounts were all in oral base per mouse.

Table 6-2 shows that collagen or LPS were orally administrated for long time to induce arthritis, and the combination of them was done to do strongly. It also shows that anti-collagen antibody and anti-bacterial-endotoxin antibody (anti-LPS antibody) in combination were administered to prevent arthritis.

**[Table 6-2]**

| g r o u p | Duration of administrating oral immunity 1 to 2 weeks, 5 to 6 weeks, 9 to 10 weeks, 13 to 14 weeks | Number of animals with arthritis occurred / number of animals administered | Max arthritis score (mean ± SD) # | Anti-chicken collagen antibody in mouse serum (U/ml) @ (number of antibody positive animals) | Induction of arthritis 16 weeks | Number of animals with arthritis occurred / number of animals induced | Arthritis (mean ± SD) |
|---|---|---|---|---|---|---|---|
| 1 | Phosphate Buffer Saline (control group) | 0/6 | 0±0 | 0 (0) | LPS lmg | 0/6 | 0±0 |
| 2 | Collagen 0.1 mg | 3/6 | 1.3±0.6* | 2.44±1.69(5) | LPS 1mg | 6/6* | 2.2±0.7* |
| 3 | Collagen 0.1 mg + anti-collagen antibody 0.1 mg | 0/6 | 0±0 | 0.04 (2) | LPS 1mg | 0/6 | 1.2±0.6 |
| 4 | LPS 0.1 mg | 4/6 | 0.6±0.6 | 0.1 (1) | LPS 1mg | 5/6* | 1.6±0.5* |
| 5 | LPS 0.1 mg + anti-LPS 0.1 mg | 0/6 | 0±0 | 0.05 (1) | LPS 1mg | 0/6 | 0±0 |
| 6 | Collagen 0.1 mg + LPS 0.1 mg | 6/6* | 1.4±0.5 | 5.5±10.0(6) | LPS 1mg | 6/6* | 3.3±0.9 ** |
| 7 | Collagen 0.1mg + LPS 0.1 mg + anti-Collagen antibody 0.1mg + anti-LPS antibody 0.1 mg | 2/6 | 0.9±0.5 | 0.6±1.0(3) | LPS 1mg | 2/6 | 0±0 |

| | | | | | | | |
|---|---|---|---|---|---|---|---|
| * Significant difference to control group, p<0.05, **: p<0.01 @Fujii K et al. J Immunol Methods 1989;124:63-70 #Terato K et al. J Exp Med 1985;162:637-46 | | | | | | | |

### Example 6-6

### Therapeutic Composition for Anti-Arthritis

A chicken was immunized with commercially available bovine Type II collagens and swine Type II collagens to get a chicken egg antibody-comprising powder having an antibody content of 50% as the total antibody amount, and a cow was immunized with chicken Type II collagen and a commercially available E. coli vaccine (imocoliv, Zenno) to get raw milk, from which a whey powder having a total antibody content of 5% was obtained. The former and latter powders were mixed at a ratio of 1: 9, and then supplied with excipients, and granulated at approximately 65°C according to a conventional method to get a granule having a total antibody content of 0.1%.

### (Method for Determining Amount of Antibody)

100 mg of functional composition comprising whey protein or antibody was dissolved in 50 ml of 50 mM phosphate buffer (pH 6. 8), stirred for 2 hours or more to get a solution, 10 ml or more of which was then filtered through a 0.45 µ-membrane filter. Meanwhile, protein G column (HiTrap Protein G HP 1 ml, Amersham) was equilibrated with 50 mM phosphate buffer (pH 6. 8), loaded with 10 ml of the sample described above which had been filtered through the membrane filter, washed with the same buffer (pH 6.8), and then loaded with 100 mM glycine-hydrochloride buffer (pH 2.7) to elute the antibody.

The absorbance of the eluate was measured at 280 nm, and then the antibody concentration of the sample was determined on the assumption that the absorbance of antibody having a concentration of 1% is 14, and then the amount of antibody in the whey protein or functional composition was calculated.

### [Reference Examples 7-1]

### (Determination of Antibody Content of Commercially Available Whey Protein)

According to the determination method described above, antibody contents of commercially available whey proteins were measured: 50.0 mg/g and 166 mg/g in Proliant 8000 (from James Farrell & Co.) and IgG-containing concentrated whey protein (Aotearoa Ltd.), respectively.

### [Examples 7-1 to 7-5, Comparative Examples 7-1 to 7-7]

4 kinds of functional composition with their compositions shown in Table 7-1 described below (numerical values represent mass ratio) were prepared. In Table 7-1, the antibody contents of the functional compositions were also shown. Further, in the following tests, Proliant 8000 was used for Comparative Examples 7-1, freeze-dried bacterial cells of Bacillus natto for Comparative Examples 7-2, dried powder of Agaricus for Comparative Examples 7-3, beer yeast powder for Comparative Examples 7-4, casein degradation product for Comparative Examples 7-5 and prolamin-treated SOD (Oxykine^{®}, from Isocell (France)) for Comparative Examples 7-6. Another two compositions of immunostimulatory substances were prepared and used for Comparative Examples 7-7.

**[Table 7-1]**

| | Example 7-1 | Example 7-2 | Example 7-3 | Example 7-4 | Example 7-5 | Comparative Example 7-7 |
|---|---|---|---|---|---|---|
| Proliant8000 | 99 | 80 | - | 90 | - | - |
| IgG-containing concentrated whey protein | - | - | 60 | - | 6 | - |
| Bucillus subtilis natto (freeze-dried fungus body) | 1 | - | - | - | - | - |
| Dried powder of agaricus | - | 20 | - | - | - | 50 |
| Beer yeast powder | - | - | 40 | - | - | 50 |
| Prolamin-treated SOD | - | - | - | 10 | - | - |
| Casein degradation product | - | - | - | - | 94 | - |
| Antibody content (mg/g) | 49.5 | 40.0 | 99.6 | 45.0 | 10.0 | - |

### [Example 7-6]

### (Effects on IgG Antibody Production)

Functional compositions prepared in Examples 7-1 to 7-5 and Comparative Examples 7-1 to 7-7 were added to prepare their respective 1% by mass mixed feeds, which were fed to allocated groups of six 6-week old BALB/c female mice to breed for two weeks. Further, mice for Control Example were fed with functional composition-free feed to breed likewise. Then, mice were immunized with 0.1 ml of physiological saline solution containing 10 µg ovalbumin and 1 mg of aluminum hydroxide, and their bloods were collected from the ocular vein plexus on the 14th day after the immunization and then serums were separated. The concentrations of anti-ovalbumin IgG antibody (anti-EAIgG) in these serums were determined as follows.

Thus, to a flat-bottomed 96-well microplate (Nunc, 439454), 50 µl of 0.1 mg/ml solution of ovalbumin in 0.05 M sodium carbonate buffer (pH 9.5) was added and left overnight at 4°C. The well was washed with a wash solution (0.9% sodium chloride, 0.05% Tween 20), supplied with 200 µl of Phosphate Buffer Saline (PBS) containing 1 mg/ml bovine serum albumin, and left to stand for an hour at 37°C for blocking. The well was washed, supplied with above serum diluted with PBS containing 10 mg /ml bovine serum albumin, 0.05% Tween 20 and 3% sodium chloride (solution A), and left to stand for an hour at 37°C. The well was washed, supplied with 50 µl of a solution of peroxidase-labeled antimouse IgG goat antibody (from Seikagaku Corporation) diluted 10000 times with solution A, left to stand for an hour at 37°C, washed, supplied with 100 µl of a substrate solution (40 mg of ortho-phenylenediamine; 20 µl of 30% hydrogen peroxide/100 ml of citric acid-sodium phosphate buffer) , and left to stand at a room temperature, to measure the absorbance at 492 nm.

A mouse was immunized with ovalbumin together with Freund' s complete adjuvant to get a serum, which was applied to ovalbumin-bound sepharose 4B affinity column to purify IgG antibody. The IgG antibody was used to make a standard curve, according to which antibody concentration was measured. The results of measured concentration of anti-EAIgG in serum (mean values of each group ± standard deviation) were shown in Table 7-2.

### [Example 7-7]

### (Effects on IgE Antibody Production)

Concentration of anti-ovalbumin IgE antibody (anti-EAIgE) in serum collected in Examples 7-6 was measured as follows. A flat-bottomed 96-well microplate well (Nunc, 439454) was supplied with 50 µl of a solution of antimouse IgE antibody in PBS, left to stand overnight at 4°C, washed with a wash solution (0.9% sodium chloride, 0. 05% Tween 20) , supplied with 200 µl of rabbit serum, left to stand for an hour at 37°C for blocking, washed, supplied the above serum diluted with PBS containing 1% rabbit serum, 0.05% Tween 20 and 3% sodium chloride (solution B), and left to stand for an hour at 37°C, washed, supplied with 50 µl of a solution of biotin-bound ovalbumin diluted with solution B, left to stand for an hour at 37°C, washed, 50 µl of a solution of peroxidase-labeled avidin (from Shigma) diluted 2500 times with solution B, and left to stand for an hour at 37°C, washed, supplied with 100 µl of a substrate solution (40 mg of ortho-phenylenediamine; 20 µl of 30% hydrogen peroxide/100 ml of citric acid-sodium phosphate buffer), left to stand at room temperature to measure the absorbance at 492 nm. The absorbance values (mean values of each group ± standard deviation) were also shown in Table 7-2. There is a positive correlation between absorbance and antibody concentration, indicating that the higher the absorbance is, the higher the antibody concentration will be.

As can be known from Table 7-2, in the Example the anti-ovalbumin IgG has a higher concentration in serum, while the anti-ovalbumin IgE has lower concentration than in Comparative Example. IgG has a role in a mechanism of protecting the body from virus, bacteria and the like, while IgE has a role in a mechanism of inducing allergic symptoms. Therefore, higher IgG and lower IgE means that Th1/Th2 balance of immune system shifts toward Th1 dominance, thus it is shown that substances in Examples 7-1 to 7-5 have more beneficial immunomodulatory effects than those in Comparative Examples 7-1 to 7-7.

**[Table 7-2]**

| | anti-EAIgG (µg/ml) | anti-EAIgE (absorbance at 492 nm) |
|---|---|---|
| Control Example | 242±87 | 0.713 ±0.203 |
| Comparative Example 7-1 | 513± 148 | 0.494 ±0.141 |
| Comparative Example 7-2 | 480± 137 | 0.543±0.163 |
| Comparative Example 7-3 | 424±126 | 0.537±0.167 |
| Comparative Example 7-4 | 446±128 | 0.584 ±0.194 |
| Comparative Example 7-5 | 491± 142 | 0.514 ±0.158 |
| Comparative Example 7-5 | 452±138 | 0.553 ±0.170 |
| Example 7-1 | 903±245 | 0.208±0.091 |
| Example 7-2 | 874±230 | 0.213±0.118 |
| Example 7-3 | 988±274 | 0.198±0.087 |
| Example 7-4 | 790±189 | 0.234±0.133 |
| Example 7-5 | 951±252 | 0.202±0.090 |
| Example 7-7 | 587±176 | 0.402±0.139 |

### [Example 7-8]

### (Effects on IL-12 production)

As subjects, fifteen men and women (9 in 21-30 years old, 3 in 31-40 years old and 3 in 41-60 years old) were divided into three groups (5 in each group). Three groups were served with 10 g/day powders of Comparative Examples 7-1 and 7-2 and Examples 7-1 respectively, which were suspended in 100 ml of water to drink after every breakfast respectively. They drank continuously for two weeks, and the induction of IL-12 production was measured before and after the continuous drinking as follows.

Namely, blood was taken from each subject using heparinized syringe, and PBMC (Peripheral Blood Mononuclear Cells) was prepared using HISTOPAQUE-1077 (from Shigma) according to a conventional method. PBMC was adjusted to 2 x 106/ml with RPMI-1640 medium which had been supplied with FCS (Fetal Calf Serum) at 10% of the medium, and every 1 ml of the solution was dispensed to each well of 24-well plate. The resultant was supplied with 0.01 KE of OK-432, cultured in CO2 incubator for 24 hours at 37°C, frozen overnight, and then thawed, and IL-12 concentration of culture supernatant was measured using human IL-12ELISA kit (from R&D Systems). The results (mean values of each group ± standard deviation) are presented in Fig. 4.

Herein, IL-12 is a cytokine "biologically active substance" secreted from macrophage and known as a extremely strong immunoreactive substance that activates natural killer cells (NK cells) and killer T cells (CTL cells), which directly attack cancer cells, and enhances the production of interferon γ (IFN-γ).

Further, OK-432 is an anti-neoplastic agent commercially available as "Picibanil" (trade name, Chugai Pharmaceutical Co. , Ltd.), and a bacterial body formulation obtained by treating strain Su of Streptococcus pyogenes (group A Type 3) in the presence of penicillin G under a certain condition and freeze-drying. OK-432 was used by Fujimoto et al. (J. Immunol.1997 Jun 15; 158 (12) : 5619-26) as a stimulating substance (trigger) in measuring immunoreactivity.

As can be known from Fig. 4, while continuous drinking composition in Comparative Examples 7-1 and 7-2 also increases IL-12 concentration, continuous drinking of composition in Examples 7-1 extremely increases IL-12 concentration compared with continuous drinking in Comparative Examples 7-1 and 7-2, suggesting immune responsiveness becoming stronger.

### [Example 7-9]

### (Examination on Intestinal Environment-Improving Effects)

As subjects, fifteen men and women (9 in 21-30 years old, 3 in 31-40 years old and 3 in 41-60 years old) were divided into three groups (5 in each group). The three groups were served with 10 g/day powders of Comparative Examples 7-1 and 7-2 and Examples 7-1 respectively, which were suspended in 100 ml of water to drink after every breakfast. They drank continuously for two weeks and answered a questionnaire survey relating to defecation in the latter one week during continuous drinking. Meanwhile, the same questionnaire survey was conducted for one week before continuous drinking. The survey items were (1) defecation frequency, (2) defecation amount, (3) color of feces and (4) smell of feces, and the results (mean values of each group ± standard deviation) are shown in Table 7-3.

About (1) defecation frequency, though drinking in Comparative Examples 7-1 and 7-2 exhibited the tendency to increase the frequency, drinking in Examples 7-1 significantly increased the frequency. About (2) defecation amount, a big difference by drinking was not shown among Comparative Examples 7-1 and 7-2 and Examples 7-1. About (3) color of feces, though drinking in Comparative Examples 7-1 and 7-2 exhibited the tendency to change the color favorable, drinking in Examples 7-1 improved the color apparently. About (4) smell of feces, though the bigger improvement was shown in Comparative Examples 7-1 than Comparative Examples 7-2, still further improvement was shown in Examples 7-1.

To summarize (1) through (4), though a little intestine-regulating effects was seen in Comparative Examples 7-1 and 7-2, drinking the composition of Examples 7-1 apparently exhibited intestine-regulating effects, thus the dominance was seen on simultaneous ingestion of antibody and fungus body.

In Table 7-3, (2) defecation amount is represented by numerical values calculated by estimating feces as the number of Ping-Pong balls having a diameter of 40 mm, (3) color of feces: yellow: 0, pale ocher: 1, ocher: 2, brown: 3, peat: 4, (4) smell of feces: almost no smell: 0, slightly smell: 1, usual smell: 2, strong smell: 3, extremely strong smell: 4.

**[Table 7-3]**

| | | Before continuous ingestion | During continuous ingestion |
|---|---|---|---|
| Defecation frequency/week | Comparative Example 7-1 | 5.6±0.8 | 6.0±0.7 |
| | Comparative Example 7-2 | 5.5±0.8 | 5.9±0.7 |
| | Example 7-1 | 5.7±0.8 | 6.8±0.6 |
| Defecation amount/week | Comparative Example 7-1 | 29.0±10.8 | 30.0±10.7 |
| | Comparative Example 7-2 | 29.5 ±10.5 | 29.9±10.7 |
| | Example 7-1 | 29.7±10.8 | 31.8±10.6 |
| Color of feces | Comparative Example 7-1 | 3.7±0.8 | 3.0±0.7 |
| | Comparative Example 7-2 | 3.5±0.8 | 2.9±0.7 |
| | Example 7-1 | 3.6±0.8 | 1.7±0.6 |
| Smell of feces | Comparative Example 7-1 | 2.8±0.6 | 1.4±0.5 |
| | Comparative Example 7-2 | 2.6±0.6 | 2.1±0.5 |
| | Example 7-1 | 2.8±0.6 | 0.4±0.3 |

### (Determination Method for Dispersibility)

Water at 15°C was poured into a 200 ml-glass beaker, a sample was added while stirring using a magnetic stirrer (SR200 supplied by Advantech Co., Ltd.) at level 3, and the period was measured from the addition of the sample to the time when the sample was uniformly dispersed.

### [Examples 8-1 to 8-5, Comparative Example 8-1]

5 kinds of powdered protein compositions with their compositions shown in Table 8-1 described below (numerical values represent mass ratio) were prepared. In Table 8-1, whey protein (WPC) used was Proliant 8000 from James Farrell & Co. Further, Avicel RC-30 is a microcrystalline cellulose supplied by Asahi Kasei Corporation, KC FLOCK W-10MG is a powder cellulose supplied by Nippon Paper Chemicals Co., Ltd., and Meioligo CR is a crystal fructo-oligosaccharide supplied by Meiji Food Materia Co., Ltd.

### (Determination of Dispersibility)

According to the method described above, results of measuring dispersibilities of powdered protein compositions prepared in Examples 8-1 to 8-5 and Comparative Example 8-1 are shown in Table 8-2. The amounts of compositions added were adjusted to have an equal amount of the protein. As seen from dispersion periods, dispersibilities were improved in Examples 8-1 to 8-5 respectively in comparison with Comparative Example 8-1.

**[Table 8 -1]**

| | Example 8-1 | Example 8-2 | Example 8-3 | Example 8-4 | Example 8-5 | Comparative Example 8-1 |
|---|---|---|---|---|---|---|
| Whey protein (WPC) | 90 | 80 | 80 | 80 | 50 | 100 |
| KC FLOCK W-10MG | 10 | - | 10 | - | 50 | - |
| Avicel RC-30 | - | 20 | - | 1 | - | - |
| Meioligo CR | - | - | 10 | 19 | - | - |

**[Table 8-2]**

| | Example 8-1 | Example 8-2 | Example 8-3 | Example 8-4 | Example 8-5 | Comparative Example 8-1 |
|---|---|---|---|---|---|---|
| Amount of composition added (g) | 1.1 | 1.1 | 1.2 | 1.2 | 2.0 | 1.0 |
| Dispersion period (min' sec) | 2' 40 | 2' 20 | 2'30 | 3' 10 | 2' 10 | 18'00 |

### [Example 8-6]

### (Granulation 1)

whey protein (WPC, powder) and KC FLOCK W-10MG were mixed in a composition shown in Example 8-1, then granulated by spray granulation, and dispersibility was measured in the same manner. The dispersion period was 2' 00", thus the dispersibility was further improved.

### [Example 8-7]

### (Granulation 2)

Using whey protein (WPC) granulated by spray granulation, a protein composition was prepared in a composition shown in Example 8-1 (KC FLOCK W-10MG is powder), and dispersibility was measured in the same manner. The dispersion period was 1'50", thus the dispersibility was further improved.

### (Method for Determining Amount of Antibody)

It was measured in the same manner as Example 4 described above.

### [Reference Example 9-1]

### (Determination of Antibody Content of Whey Protein)

Similar to Reference Example 4-1.

### [Examples 9-1 to 9-5]

Similar to Examples 4-1 to 4-5 and Table 4-1.

Evaluating improvement of flavor is Similar to

### Example 4 and Table 4-2.

### [Examples 9-6 to 9-8]

Similar to Examples 4-6 to 4-8.

### [Example 9-9]

Similar to Example 4-9.

### [Example 9-10]

Similar to Example 4-10.

### [Example 9-11]

Similar to Example 4-11.

### [Example 9-12]

Similar to Example 4-12.

### [Example 9-13 to 9-20, Comparative Example 9-2]

In the following Examples, water absorption was measured by the method below.

### (Measurement for Water Absorption)

Cow milk at 5°C was poured into a 100 ml-glass beaker, and a sample was added to contain 2.5 g of whey protein while stirring using a magnetic stirrer (SR200 supplied by Advantech Co., Ltd.) at level 3, and the period was measured from addition of the sample to the time when sample was uniformly dispersed.

5 kinds of whey protein foods having their compositions shown in Table 9-3 described below (numerical values represent mass ratio) were prepared. In Table 9-3, as WPC granule, Proliant 8000 was granulated (mean granule size of approximately 0.2 mm) by spray granulation and used. Water absorption of the obtained whey protein food and granular (mean granule size of approximately 0.2 mm) whey protein food (Example 9-18 to 9-20) used in Examples 9-6 to 9-8 was measured respectively according to the method described above. The results are shown in Table 9-4 and 9-5.

**[Table 9-3]**

| | Comparative Example 9-2 | Example 9-13 | Example 9-14 | Example 9-15 | Example 9-16 | Example 9-17 |
|---|---|---|---|---|---|---|
| WPC granule | 100 | 76 | 50 | 25 | 50 | 50 |
| Meioligo P | - | 24 | 50 | 75 | - | - |
| Oligomate 55P | - | - | - | - | 50 | |
| Sucrose | - | - | - | - | - | 50 |

**[Table 9-4]**

| | Comparative Example 9-2 | Example 9-13 | Example 9-14 | Example 9-15 | Example 9-16 |
|---|---|---|---|---|---|
| Dispersion period (min'sec) | 30'00 | 4'20 | 3'30 | 2'50 | 3'50 |

**[Table 9-5]**

| | Example 9-17 | Example 9-18 | Example 9-19 | Example 9-20 |
|---|---|---|---|---|
| Dispersion period (min'sec) | 7'00 | 10'20 | 9'50 | 9'00 |

As shown in Tables 9-4 and 9-5, addition of powder saccharide to WPC granule improved water absorption more than granule of WPC alone and the mixed granule of WPC and glucide did.

### [Example 10-1, Comparative Examples 10-1 and 10-2]

3 kinds of whey protein foods with their compositions (numerical values represent mass ratio) shown in Table 10-1 described below were prepared. In Table 1, Proliant^{®} 8000 is a whey protein (WPC, from James Farrell & Co.) and Meioligo^{®} P is fructo-oligosaccharide (from Meiji Seika Kaisha, Ltd.) .

**[Table 10-1]**

| | Example 10-1 | Comparative Example 10-1 | Comparative Example 10-2 |
|---|---|---|---|
| Proliant8000 | 90 | 90 | 90 |
| Meioligo P | 5 | 10 | - |
| Pectin | 5 | - | 10 |

### (Determination Method for Bifidobacteria and Clostridium perfringens)

Counting the number of bifidobacteria: After sterilization, BL agar medium "Nissui" (Nissui Pharmaceutical Co. , Ltd) was cooled to 50°C, supplied with an addition solution for BS culture medium (30% sodium propionate, 6% lithium chloride, 0.1% Paromomycin sulfate, 0.1% Neomycin sulfate) by 5% and horse defibrinated blood by 5%, and dispensed into a dish to prepare a plate. Diluted sample was applied there with Conradi stick and cultured anaerobically for 20 hours at 37°C and counted.

Counting the number of Clostridium perfringens: CW agar base medium "Nissui" (Nissui Pharmaceutical Co. , Ltd.) containing kanamycin was sterilized, dispensed into a dish to prepare a plate. Diluted sample was applied there with Conradi stick, and cultured anaerobically for 20 hours at 37° and counted.

Three subjects were served each 10 g/day of powders of Example 10-1, Comparative Examples 10-1 and 10-2 according to the order presented in Table 10-2 through each two-week period (total six weeks), which was suspended in 100 ml of water to drink after every breakfast. During each two-week period, feces were sampled in the latter one week. Meanwhile, feces were also sampled for a week before the test started. The numbers of bifidobacteria and Clostridium perfringens in these feces were counted by the method described above, and values in each test section were averaged. These measurement results are put into a graph shown in Fig. 5.

### Table 10-2

### Drinking Order

Subject A Example 10-1→ Comparative Example 10-1→ Comparative Example 10-2
Subject B Comparative Example 10-1→ Comparative Example 10-2→ Example 10-2
Subject C Comparative Example 10-2→ Example 10-1→ Comparative Example 10-1

As shown in Fig. 5, the whey protein food of Comparative Examples 10-1 and 10-2 was ingested to increase a little the bifidobacteria of good bacterium compared with those before the test started, while the whey protein food of Example 10-1 was ingested to increase extremely. Meanwhile, Clostridium perfringens of bad bacteria decreased after ingesting food of Comparative Examples 10-1 and 10-2 compared with those before the test started, and further decreased after ingesting the food of Example 10-1.

From these facts, it was demonstrated that by intaking whey protein food shown in Example 10-1, intestinal environment was greatly improved as compared with those of Comparative Examples 10-1 and 10-2.

### [Example 10-2]

### (Examination of Intestinal Environment-Improving Effects 1)

As subjects, fifteen men and women (9 in 21-30 years old, 3 in 31-40 years old and 3 in 41-60 years old) were divided into three groups (5 in each group). The three groups were served with 10 g/day powders of whey protein food of Example 10-1 and Comparative Examples 10-1 and 10-2 respectively, which were suspended in 100 ml of water to drink after every breakfast. They drank continuously for two weeks and answered a questionnaire survey relating to defecation in the latter one week during continuous drinking. Meanwhile, the same questionnaire survey was conducted for one week before continuous drinking. The survey items were (1.) defecation frequency, (2) defecation amount, (3) color of feces and (4) smell of feces, and the results (mean values of each group ± standard deviation) are shown in Table 10-3.

About (1) defecation frequency, though drinking the powders of whey protein food of Comparative Examples 10-1 and 10-2 exhibited the tendency to increase the frequency, drinking the powder of Example 10-1 significantly increased the frequency. About (2) defecation amount, a big difference by drinking was not shown among foods of Comparative Examples 10-1 and 10-2, and Example 10-1. About (3) color of feces, though taking the food of Comparative Examples 10-1 and 10-2 exhibited the tendency to change the color favorable, drinking those of Example 10-1 improved the color apparently. About (4) smell of feces, though improvements were shown in Comparative Examples 10-1 and 10-2, still further improvement was shown in Example 10 -1.

To summarize (1) through (4), though a little intestine-regulating effect was seen in whey protein foods of Comparative Examples 10-1 and 10-2, drinking the whey protein food of Example 10-1 apparently exhibited intestine-regulating effect, thus the dominance was seen on simultaneous ingestion of three substances of antibody, oligosaccharide and dietary fiber.

In Table 10-3, numerical values mean the following. (3) color of feces; 0: yellow, 1: pale ocher, 2: ocher, 3: brown, 4: peat,
(4) smell of feces; 0: almost no smell, 1: slight smell, 2: usual smell, 3: strong smell, 4: extremely strong smell

**[Table 10-3]**

| | | Before continuous ingestion | During continuous ingestion |
|---|---|---|---|
| Defecation frequency/week | Comparative Example 10-1 | 5.7±0.8 | 6.1±0.6 |
| | Comparative Example 10-2 | 5.5±0.8 | 5.9±0.7 |
| | Example 10-1 | 5.8±0.8 | 6.8±0.6 |
| Defecation amount/week | Comparative Example 10-1 | 28.7±10.6 | 30.6±10.7 |
| | Comparative Example 10-2 | 29.3±10.5 | 29.8±10.7 |
| | Example 10-1 | 29.6±10.8 | 30.9±10.6 |
| Color of feces | Comparative Example 10-1 | 3.5±0.8 | 2.8±0.6 |
| | Comparative Example 10-2 | 3.7±0.8 | 3.0±0.7 |
| | Example 10-1 | 3.6±0.8 | 1.5±0.5 |
| Smell of feces | Comparative Example 10-1 | 2.8±0.6 | 1.5±0.4 |
| | Comparative Example 10-2 | 2.6±0.6 | 1.6±0.5 |
| | Example 10-1 | 2.7±0.6 | 0.4±0.3 |

### [Example 10-3]

### (Examination of Intestinal Environment-Improving Effects 2)

Whey protein foods were prepared in the same way as in Example 10-1 and Comparative Example 10-1 but lactulose was used in place of Meioligo P, and tested similarly as in Example 10-2. Results similar to Example 10-2 were obtained, thus the dominance was seen on simultaneous ingestion of the three substances of antibody, oligosaccharide and dietary fiber.

### [Example 10-4]

### (Examination of Intestinal Environment-Improving Effects 3)

Whey protein foods were prepared in the same way as in Example 10-1 and Comparative Example 10-1 but trehalose was used in place of Meioligo P, and tested similarly as in Example 10-2. Results similar to Example 10-2 were obtained, thus the dominance was seen on simultaneous ingestion of the three substances of antibody, oligosaccharide and dietary fiber.

### [Example 10-5]

### (Examination of Intestinal Environment-Improving Effects 4)

Whey protein foods were prepared in the same way as in Example 10-1 and Comparative Example 10-2 but sodium arginate was used in place of pectin, and tested similarly as in Example 10-2. Results similar to Example 10-2 were obtained, thus the dominance was seen on simultaneous ingestion of the three substances of antibody, oligosaccharide and dietary fiber.

### [Example 10-6]

### (Examination of Intestinal Environment-Improving Effects 5)

Whey protein foods were prepared in the same way as in Example 10-1 and Comparative Example 10-2 but glucomannan was used in place of pectin, and tested similarly as in Example 10-2. Results similar to Example 10-2 were obtained, thus the dominance was seen on simultaneous ingestion of the three substances of antibody, oligosaccharide and dietary fiber.

### (Method for Determining Amount of Antibody)

An anti-bovine IgG rabbit immune serum (from YAGAI Corporation) was applied on protein G column to fractionate an adsorbed fraction. The fraction was used as primary antibody, and HRP labeled anti-bovine IgG antibody (from Cosmo Bio Co., Ltd.) was used as secondary antibody, to determine by ELISA according to a common method.

### (Method for Counting E. coli and Coliform groups)

Measurement was conducted using ES Colimark agar medium (from Eikenkizai Co., Ltd.) .

### (Method for Counting Staphylococcus aureus)

Salt egg york agar base medium (from Nissui K. K.) was used.

### (Method for identifying Listeria monocytogenes)

The bacteria was directly cultured in Oxford agar medium (Difco) to separate, and identification test was conducted on colonies generated.

### [Reference Example 11-1]

### (Separation of Antibody)

Cow's colostrum was centrifuged to remove fat, supplied with hydrochloric acid to adjust pH to 4.6 for precipitating casein to remove. The supernatant was supplied with Sodium hydroxide to adjust pH to 7.0, and freeze-dried to obtain colostrum whey powder. This colostrum whey powder was dissolved again, adsorbed/desorbed thrice on protein G column according to a conventional method, and dialyzed to remove a low molecular fraction. The protein G-adsorbed fraction thus obtained was used as the authentic product of antibody in cow milk. This authentic product was used to depict a standard curve, which was used to determine the antibody concentration of the sample.

### [Example 11-1]

### (Confirming decolonization by lactobacilli fermentation 1)

The amount of antibody of raw milk obtained was determined by the ELISA to contain 151 µg/ml. The number of Coliform groups was 20/ml and that of Staphylococcus aureus was 50/ml. 1L of this raw milk was supplied with 50 ml of precultured lactobacilli (Lactobacillus casei) starter and fermented for 15 hours at 37°C to obtain a yogurt having a good flavor. The amount of antibody in this yogurt was determined to contain 144 µg/ml, showing no antibody lost during fermentation. Further, both numbers of Coliform groups and Staphylococcus aureus were 10/ml or less (less than detection limit), thus it was confirmed that Coliform groups and Staphylococcus aureus were eliminated by lactobacilli.

Meanwhile, when raw milk was left to stand for 15 hours at 37°C without lactobacilli starter supplied, the number of Coliform groups was 1.1 x 107/ml and Staphylococcus aureus was 2.3 x 106/ml.

### [Example 11-2]

### (Confirming Decolonization by Lactobacilli Fermentation 2)

The same raw milk as used in Example 11-1 was supplied with E. coli to be 50/ml. 1L of this E. coli-supplied raw milk was supplied with 88% lactic acid solution (from Boegan trading Co., Ltd.) to adjust pH to 5.8, and further supplied with glycerin by 1% of the total. The resultant was supplied with 50 ml of precultured lactobacilli (Lactobacillus reuteri) starter and fermented for 24 hours at 37°C, to reveal that the numbers of the E. coli, the Coliform groups and the Staphylococcus aureus fell down to 10/ml or less (less than detection limit). Thus it was confirmed that these bacteria were eliminated by the lactobacilli. Further, this yogurt was rather acidic but had good flavor.

### [Example 11-3]

### (Confirming decolonization by lactobacilli fermentation 3)

Another raw milk obtained was analyzed on Listeria monocytogenes, showing that it was positive. 1L of this raw milk was supplied with each 20 ml of precultured lactobacilli (Enterococcusfaecalis) and of Bifidobacteria bifidum starters and fermented for 20 hours at 37°C, showing that it became Listeria monocytogene-negative. Further, the numbers of E. coli, Coliform groups and Staphylococcus aureus became 10/ml or less (less than detection limit), thus it was confirmed that these bacteria were terminated.

### [Example 11-4]

Each 1L of Ultra high temperature sterilized (130°C for 2 seconds) cow milk, low temperature sterilized (66°C for 30 minutes) cow milk and non-sterilized raw milk was supplied with 20 ml of precultured lactobacilli (Lactobacillus casei) starter respectively and fermented for 15 hours at 37°C to obtain their respective yogurts. They were cooled in a refrigerator and tasted by 10 women in their twenties. The tastiest one was selected by them respectively and their comments on taste and flavor were obtained. The results were as follows.

### (The most favored one and number of persons who selected it)

Yogurt made from ultra high temperature sterilized cow milk (A) 0 person
Yogurt made from low temperature sterilized cow milk

### (B) 1 person

Yogurt made from non-sterilization cow milk (C) 9 persons
(comments on each yogurt)
(A) : Strongly acidic feeling. Rather burning smell. No rich taste.
(B): Rather watery and dull feeling. Mild taste.
(C) : Rich and slightly sweet taste. Unique and fresh flavor.

### (Method for Determining Amount of Antibody)

The anti-bovine IgG rabbit immune serum (from YAGAI Corporation) was applied on protein G column to get an adsorbed fraction. The adsorbed fraction was used as primary antibody, and HRP labeled anti-bovine IgG antibody (from Cosmo Bio Co., Ltd.) was used as secondary antibody to determine an antibody amount by ELISA according to a common method. Whey powder obtained from cow's colostrum was adsorbed/desorbed thrice on protein G column according to a conventional method to get a protein G-adsorbed fraction as an antibody preparation. The fraction was dialyzed to remove a low molecular fraction and used to prepare a standard curve for determining the antibody concentration of the sample.

### [Reference Example 12-1]

### (Determination of Amount of Antibody in Whey Protein)

According to the determination method described above, the amount of antibody in a commercially available whey protein was determined to give as follows.
WPC (Whey Protein Concentrate): 3.9%
WPI (Whey Protein Isolate): 1.1%
IgG-containing concentrated whey protein: 14.5%

### [Example 12-1]

### (Whey Protein-Containing Fermented Food 1)

1 L of commercially available ultra high temperature sterilized cow milk (130°C for 2 seconds) was supplied with 50 g of Sunlact N-5 (supplied by Taiyo Kagaku Co., Ltd.) of whey protein, and further supplied with 50 ml of precultured lactobacilli (Lactobacillus casei) starter, and fermented for 15 hours at 37°C to obtain a yogurt having a good flavor. 1 g of this yogurt contained 1.6 mg of antibody and 8 x 109 lactobacilli.

### [Example 12-2]

### (Whey Protein-Containing Fermented food 2)

1 L of commercially available soy milk preparation was supplied with 100 g of Milactele 80 (from Morinaga Milk Industry Co., Ltd.) of whey protein, and then supplied with 50 ml of precultured lactobacilli (Lactobacillus acidphilus) starter, and fermented for 15 hours at 37°C to obtain a yogurt-like soy milk fermented food. 1 g of this soy milk fermented food contained 3.4 mg of antibody and 2 x 10⁷ lactobacilli.

For comparison, 1 L of soy milk preparation was supplied with 50 ml of precultured lactobacilli (Lactobacillus acidphilus) starter and no whey protein, and fermented for 24 hours at 37°C. Compared with a product fermented with whey protein supplied, it was difficult to solidify was released plenty of water. Further, no antibody was detected.

### [Example 12-3]

### (Whey Protein-Containing Fermented Food 3)

1 L of commercially available ultra high temperature sterilized cow milk was supplied with 50 ml of precultured lactobacilli (Lactobacillus gasseri) starter, and fermented for 15 hours at 37°C. This fermented solution was supplied with 25 g of milk-IgG-containing concentrated whey protein (Aotearoa Ltd.) and 20 ml of Agaricus extract, and efficiently stirred to obtain a yogurt beverage. 1 g of this yogurt beverage contained 3.3 mg of antibody and 4 x 109 lactobacilli.

### [Example 12-4]

### (Whey Protein-Containing Nonfermented Food)

Bifidobacteria (Bifidobacteria longum) was cultured with nonfat milk with 0.2% yeast extract added. This culture medium was cooled to 5°C to get a nonfermented solution. 50 ml of the solution was mixed with 950 ml of commercially available ultra high temperature sterilized cow milk which had been cooled to 5°C, and then supplied with 25 g of milk-IgG-containing concentrated whey protein (Aotearoa Ltd.), and efficiently stirred to obtain a nonfermented beverage. 1 g of this nonfermented beveragecontained 3.5 mg of antibody and 3 x 10⁷ bifidobacteria.

### [Example 12-5]

### (Colostrum-Containing Fermented Food)

0.9 L of usual raw milk and 0.1 L of cow's pooled colostrum were mixed, and supplied with 50 ml of precultured lactobacilli (Lactobacillus casei) starter, and fermented for 15 hours at 37°C to obtain a yogurt. 1 g of this yogurt contained 1.7 mg of antibody and 9 x 108 lactobacilli.

### (Method for Determining Amount of Antibody)

The anti-bovine IgG rabbit immune serum (from YAGAI Corporation) was applied on protein G column to get an adsorbed fraction. The adsorbed fraction was used as primary antibody, and HRP labeled anti-bovine IgG antibody (from Cosmo Bio Co., Ltd.) was used as secondary antibody to determine an antibody amount by ELISA according to a common method. Whey powder obtained from cow's colostrum was adsorbed/desorbed thrice on protein G column according to a conventional method to get a protein G-adsorbed fraction as an antibody preparation. The fraction was dialyzed to remove a low molecular fraction and used to prepare a standard curve for determining the antibody concentration of the sample.

### [Reference Example 13-1]

### (Determination of Antibody Content of Whey Protein)

According to the determination method described above, antibody contents of commercially available whey proteins were determined: Sunlact N-5 (Taiyo Kagaku Co., Ltd.), Milacteale 80 (Morinaga Milk Industry Co., Ltd.), ALACEN 392 (Fonterra Japan K. K.) and TATUA 902 (Tatua Japan K. K.) had their respective antibody contents of 3.9% by mass, 3.9% by mass, 3.7% by mass and 3.8% by mass. Further, antibody content of milk TgG-containing concentrated whey protein (Aotearoa Ltd.) was 15.5% by mass.

### [Example 13-1]

### (Antibody-containing processed Milk)

1 L of commercially available ultra high temperature sterilized cow milk was supplied with 80 g of milk-IgG-containing concentrated whey protein (Aotearoa Ltd.) and 20 g of fructo-oligosaccharide, and efficiently stirred. At that time, glucide content was 6.2% by mass. It was heated to sterilize for 30 minutes at 65°C to obtain an antibody-containing processed milk. 1 g of this antibody-containing processed milk contained 10.4 mg of antibody derived from milk, and the percentage of remaining antibody was 90%.

### [Example 13-2]

### (Antibody Containing Vegetable Juice Beverage)

To 400 g of carrot juice, 100 g of spinach juice, 300 g of tomato juice, 150 g of apple juice, and 30 g of milk-IgG-containing concentrated whey protein (Aotearoa Ltd.) was supplied with 20 g of Agaricus extract as the other immunostimulatory component, and mixed. Glucide content at that time was 4.3% by mass. It was heated to sterilize for 30 minutes at 60°C to obtain an antibody containing vegetable juice beverage. 1 g of this antibody containing vegetable juice beverage contained 2.4 mg of antibody derived from milk, and the percentage of remaining antibody was 52%.

### [Example 13-3]

### (antibody containing-soy beverage)

1 L of a commercially available soy beverage was supplied with 100 g of Sunlact N-5 (Taiyo Kagaku Co., Ltd.), and efficiently stirred, thereby glucide content being 4.5% by mass. It was heated to sterilize for 30 minutes at 65°C to obtain an antibody containing-soy beverage. 1 g of this antibody containing-soy beverage contained 3.2 mg of antibody derived from milk, and the percentage of remaining antibody was 89%.

### [Example 13-4]

### (Antibody-Containing Chocolate)

900 g of fragmented chocolate (glucide contain of 56% by mass) was heated to 90°C to melt. It was cooled to reach a product temperature of 70°C, and supplied with 100 g of Milactele 80 (Morinaga Milk Industry Co., Ltd.) of whey protein, and homogenized to obtain an antibody-containing chocolate. 1 g of this antibody-containing chocolate contained 3.6 mg of antibody derived from milk, and the percentage of remaining antibody was 93%.

### [Example 13-5]

### (Antibody-Containing Ice Cream)

Two egg yolks and 40 g of granulated sugar were efficiently mixed, and then supplied with 150 ml fresh cream, 50 ml of commercially available ultra high temperature sterilized cow milk, 50 ml of water, and 15 g of ALACEN 392 (Fonterra Japan K. K.) of whey protein, and efficiently stirred. Glucide content at that time was 13% by mass. It was heated to sterilize for 30 minutes at 60°C, supplied uniformly with a little vanilla essence, cooled, and then set into Ice Cream Maker (Toshiba Corporation). 1 g of resulting antibody-containing ice cream contained 1.7 mg of antibody derived from milk, and the percentage of remaining antibody was 97%.

### [Example 13-6]

### (Antibody-Containing Jelly)

200 ml of a commercially available ultra high temperature sterilized cowmilk was heated to 60°C, supplied with 50g of sugar and 5 g of gelatin which had been swollen with a small amount of water, and was heated to 95°C to dissolve completely. It was cooled to 60°C, supplied with 25 g of TATUA 902 (Tatua Japan K. K.) of whey protein, quickly stirred with no form whipped up, and then poured into a jelly container. Glucide content at that time was 20% by mass. Then, it was kept at 60°C for 30 minutes to sterilize and then cooled to 5°C to obtain an antibody-containing jelly. 1 g of this antibody-containing jelly contained 3.2 mg of antibody derived from milk, and the percentage of remaining antibody was 92%.

### [Example 13-7]

### (Antibody-Containing Processed Cheese)

1 kg of natural cheese (cheddar cheese) was shredded, then supplied with 30 g of sodium polyphosphate and 100 g of water, and heated to 80°C for 10 minutes to melt. It was cooled to reach a product temperature of 65°C, supplied with 100 g of Sunlact N-5 (Taiyo Kagaku Co., Ltd.) of whey protein, efficiently kneaded, and then placed in a container to mold. Glucide content at that time was 1.4% by mass. It was heated to sterilize for 30 minutes at 60°C to obtain an antibody-containing processed cheese. 1 g of this antibody-containing processed cheese contained 3.2 mg of antibody derived from milk, and the percentage of remaining antibody was 96%.

### [Example 13-8]

### (Antibody-containing Cocoa Beverage)

40 g of cocoa powder, 70 g of sugar, 25 g of erythritol, 120 g of Milactele 80 (Morinaga Milk Industry Co., Ltd.) of whey protein were dissolved in 1 L of water. Glucide content at that time was 7.6% by mass. It was heated to sterilize for 30 minutes at 70°C to obtain an antibody-containing cocoa beverage. 1 g of this antibody-containing cocoa beverage contained 1.9 mg of antibody derived from milk, and the percentage of remaining antibody was 52%.

### [Example 14-1 to 14-6]

Six kinds of whey protein foods having their respective compositions shown in Table 1 described below were prepared. In Table 14-1, Proliant® 8000 is a whey protein (from WPC, James Farrell & Co.). Further, the followings were used respectively: green tea powder supplied by Shimamoto K. K., aloe powder of "candelabra aloe powder" by Aloe Shokuhin K. K., turmeric powder of "autumn turmeric powder" by K. K. Okinawa Ichiba, pumpkin powder from Tennen Sozai K. K., red grape juice powder by Asama chemical Co., Ltd., and tomato powder Sunbright Co., Ltd.

**[Table 14-1]**

| | Comparative Example | Example 14-1 | Example 14-2 | Example 14-3 | Example 14-4 | Example 14-5 | Example 14-6 |
|---|---|---|---|---|---|---|---|
| Proliant 8000 | 100 | 80 | 50 | 99 | 90 | 90 | 10 |
| Green tea powder | - | 20 | - | - | - | - | - |
| Aloe powder | - | - | 50 | - | - | - | - |
| Turmeric powder | - | - | - | 1 | - | - | - |
| Pumpkin powder | - | - | - | - | 10 | - | - |
| Red grape juice powder | - | - | - | - | - | 10 | - |
| Tomato powder | - | - | - | - | - | - | 90 |

| | | | | | | | |
|---|---|---|---|---|---|---|---|
| (Evaluating Improvement of Flavor) | | | | | | | |

5 parts by mass of each whey protein food prepared was homogenously added to 95 parts by mass of a commercially available plain yogurt to get one product, and similarly homogenously added to 95 parts by mass of a commercially available ultra high temperature sterilized cow milk (hereinafter, abbreviated as "cow milk") to get another product. The two products were subjected to sensory evaluation by 10 expert panelists according to 5-point scale. Their average scores are shown in Table 14-2. A higher score means better flavor. The Examples all acquired high scores, thus six kinds of whey protein foods all were improved in the flavor of whey protein.

**[Table 14-2]**

| | Comparative Example | Example 14-1 | Example 14-2 | Example 14-3 | Example 14-4 | Example 14-5 | Example 14-6 |
|---|---|---|---|---|---|---|---|
| Flavor of yogurt | 1.6 | 4.6 | 4.4 | 4.3 | 4.1 | 4.4 | 3.5 |
| Flavor of cow milk | 1.4 | 4.3 | 4.1 | 4.2 | 3.9 | 4.2 | 3.6 |

Meanwhile, in this Example, whey protein foods was supplied with green tea, aloe, turmeric, pumpkin, red grape juice and tomato powders respectively, and added to yogurt to drink. They all hardly gave a "floating mass" compared with a powder containing whey protein alone, indicating that they were improved in water absorption.

### [Example 14-7 to 14-8]

Whey protein foods having their respective compositions shown in Examples 14-1 and 14-3 were granulated into granular whey protein food by spray granulation. When 5 parts by mass of those granular whey protein foods were added to 95 parts by mass of cow milk, they quickly absorbed water, precipitated in the cow milk and dispersed.

Example 15 was similar to Example 4.

## Claims

1. A functional composition comprising an antibody and an immunostimulatory substance as effective components.

2. The functional composition according to claim 1, wherein the antibody is an antibody derived from milk.

3. The functional composition according to claim 1 or 2, wherein the immunostimulatory substance is a bacterial body or components of a bacterium which produces no toxin, excluding lactobacilli and bifidobacteria.

4. The functional composition according to claim 1 or 2, wherein the immunostimulatory substance is a fungus body or fungal components of a mold/yeast.

5. The functional composition according to claim 1 or 2, wherein the immunostimulatory substance is a mushroom or components of a mushroom.

6. The functional composition according to claim 1 or 2, wherein the immunostimulatory substance is an enzymatic degradation product of materials selected from bacteria, molds/yeasts and mushrooms.

7. The functional composition according to claim 1 or 2, wherein the immunostimulatory substance is SOD.

8. A composition for treating rheumatoid arthritis comprising an antibody having antirheumatic effect as an effective component.

9. The composition for treating rheumatoid arthritis according to claim 8, wherein the antibody having antirheumatic effects is an anti-Type II collagen antibody.

10. The composition for treating rheumatoid arthritis according to claim 9, wherein the anti-Type II collagen antibody is at least any of anti-chicken Type II collagen antibody, anti-bovine Type II collagen antibody, and anti-porcine Type II collagen antibody.

11. The composition for treating rheumatoid arthritis according to claim 8, wherein the antibody having antirheumatic effect is an anti-bacterial-endotoxin antibody.

12. The composition for treating rheumatoid arthritis according to claim 8, wherein the antibody having antirheumatic effect is obtained by mixing an anti-Type II collagen antibody and an anti-bacterial-endotoxin antibody in an appropriate ratio.

13. The composition for treating rheumatoid arthritis according to claim 8, comprising at least either anti-Type II collagen antibody or anti-bacterial-endotoxin antibody at 0.1% by mass or more as a total amount of the antibody.

14. A method for producing an antibody-containing fermented food, wherein raw milk of a cow or a mammal beside the cow is exempted from heating at 63°C or more to be supplied with one or more of lactobacilli or bifidobacteria and fermented.

15. The method for producing an antibody-containing fermented food according to claim 14, wherein the raw milk is supplied with an organic acid to adjust the pH to 5.8 or less, followed by addition of one or more of lactobacilli or bifidobacteria thereto.

16. The method for producing an antibody-comprising fermented food according to Claim 14 or 15, wherein the above described raw milk is defatted and fermented.

17. An antibody-containing fermented food obtained by the production method according to any of claims 14 to 16.

18. An antibody-containing food comprising 1.5 mg/g or more of antibody and 10⁷/g or more of one or more bacteria selected from the group consisting of lactobacilli and bifidobacteria.

19. The antibody-containing food according to claim 18, comprising 3 mg/g or more of the antibody.

20. The antibody-containing food according to claim 18 or 19, wherein the antibody is an antibody contained in whey protein.

21. The antibody-containing food according to any one of claims 18 to 20, wherein the antibody is a colostral antibody.

22. The antibody-containing food according to any one of claims 18 to 21, further comprising another component having an immunostimulatory function.

23. An antibody-containing apparent food, comprising 1. 5 mg/g or more of antibody derived from milk, with the proviso that the food does not contain lactobacilli and bifidobacteria at 10⁷/g or more.

24. The antibody-containing apparent food according to claim 23, comprising 3 mg/g or more of the antibody derived from milk.

25. The antibody-containing apparent food according to claim 23, comprising 10 mg/g or more of the antibody derived from milk.

26. The antibody-containing apparent food according to any one of claims 23 to 25, wherein the antibody derived from milk is an antibody contained in whey protein.

27. The antibody-containing apparent food according to any one of claims 23 to 26, further comprising a component having an immunostimulatory function, excluding the antibody derived from milk.

28. The antibody-containing apparent food according to any one of claims 23 to 27, wherein the antibody-containing apparent food is a dairy product or frozen dessert.

29. A method for producing the antibody-containing apparent food according to claim 28, wherein the apparent food containing the antibody derived from milk or a portion containing the antibody derived from milk in the apparent food is heated to sterilize under a condition allowing 50% or more of the antibody to remain.

30. A method for producing the antibody-containing apparent food according to claim 29, wherein the antibody-containing portion of the food is adjusted to have a glucide content of 7.5% by mass or more and then heated to sterilize.

31. A whey protein food comprising whey protein and glucide, cocoa powder, powdery unsolidifiable and insoluble substance, green tea, aloe, turmeric, pumpkin, red grape juice, tomato, cranberry, raspberry, blueberry or strawberry powder.

32. The whey protein food according to claim 31, comprising whey protein and 5 to 1,000 parts by mass of glucide based on 100 parts by mass of the whey protein.

33. The whey protein food according to claim 32, wherein the glucide is one or more indigestible saccharides.

34. The whey protein food according to claim 32, wherein the indigestible saccharide is selected from fructo-oligosaccharide, lactosucrose, lactulose, isomalt-oligosaccharide and galacto-oligosaccharide with no α-galactosyl bond contained.

35. The whey protein food according to claim 31, comprising whey protein and 5 to 900 parts by mass of one or more oligosaccharides selected from fructo-oligosaccharide, lactosucrose, lactulose, isomalt-oligosaccharide and galacto-oligosaccharide with no having α-galactosyl bond contained based on 100 parts by mass of the whey protein.

36. The whey protein food according to any one of claims 32 to 34, having a granular form.

37. The whey protein food according to any of claims 32 to 34, wherein the content of antibody or antibody derived from milk is 5 mg/g or more.

38. The whey protein food according to any of claims 32 to 34, wherein the content of antibody or antibody derived from milk is 10 mg/g or more.

39. The whey protein food according to any of claims 32 to 34, wherein the content of antibody or antibody derived from milk is 50 mg/g or more.

40. The whey protein food according to any of claims 32 to 34, wherein the whey protein is a granular and the glucide is a powder.

41. The whey protein food according to any of claims 32 to 34, wherein the glucide is contained at 30 parts by mass or more based on 100 parts by mass of the whey protein.

42. A fecal odor-reducing agent comprising whey protein.

43. The fecal odor-reducing agent according to claim 42, wherein the whey protein contains an antibody an effective component.

44. The fecal odor-reducing agent according to claim 42 or 43, wherein the content of the antibody is 0.5% by mass or more.

45. The fecal odor-reducing agent according to any one of claims 42 to 44, further comprising an indigestible saccharide.

46. The whey protein food according to claim 31, comprising the whey protein and 1 to 10,000 parts by mass of cocoa powder based on 100 parts by mass of the whey protein.

47. The whey protein food according to claim 46, having a granular form.

48. The whey protein food according to claim 31, comprising whey protein and 1 to 900 parts by mass of one or more powders selected from cranberry, raspberry, blueberry and strawberry powders based on 100 parts by mass of the whey protein.

49. The whey protein food according to claim 48, having a granular form.

50. A protein composition excellent in water dispersibility comprising a powdered or granulated protein derived from milk and a powdery unsolidifiable and insoluble substance.

51. The protein composition according to claim 50, wherein the powdery unsolidifiable and insoluble substance is cellulose.

52. The protein composition according to claim 50 or 51, wherein the powdered or granulated protein derived from milk is whey protein.

53. The protein composition according to any one of claims 50 to 52, further comprising an indigestible saccharide.

54. A whey protein food, comprising whey protein, oligosaccharide and a water-soluble dietary fiber.

55. The whey protein food according to claim 54, having a granular form.

56. The whey protein food according to claim 31, comprising whey protein and 1 to 900 parts by mass of a powder selected from green tea, aloe, turmeric, pumpkin, red grape juice and tomato powders based on 100 parts by mass of the whey protein.

57. The whey protein food according to claim 56, having a granular form.

58. A method for producing whey protein, wherein glucide is added at least any time during the production process of the whey protein to increase the percentage of remaining antibody.

59. The method for producing whey protein according to claim 58, wherein the glucide is added before raw milk is heated to sterilize.

60. The method for producing whey protein according to claim 58, wherein the glucide is added before the whey is heated.

61. The method for producing whey protein according to claim 59 or 60, wherein the glucide is lactose.

62. The method for producing whey protein according to claim 58, wherein the glucide is added before the whey protein is dried.

63. The method for producing whey protein according to claim 62, wherein the glucide is an indigestible saccharide.

64. The method for producing whey protein according to any of claims 58 to 63, wherein the percentage of remaining antibody is of 70% or more of antibody contained in raw material milk.
